# EUROPÄISCHE PATENTSCHRIFT

(11) **EP 2 621 713 B1**
(45) Veröffentlichungstag und Bekanntmachung des Hinweises auf die Patenterteilung: **23.09.2015**
(21) Anmeldenummer: 11764486.4
(22) Anmeldetag: 30.09.2011
(51) Int. Cl.: C12N 5/071, B29C 67/00, C12N 5/00

(54) **VORRICHTUNG UND VERFAHREN ZUR SCHICHTWEISEN HERSTELLUNG VON 3D-STRUKTUREN, SOWIE DEREN VERWENDUNG**
DEVICE AND METHOD FOR THE PRODUCTION OF 3D STRUCTURES IN LAYERS, AND USE THEREOF
PROCÉDÉ ET DISPOSITIF DE FABRICATION EN COUCHES DE STRUCTURES TRIDIMENSIONNELLES ET UTILISATION DESDITS PROCÉDÉ ET DISPOSITIF

(30) Priorität: 25.02.2011 DE 102011012412; 01.10.2010 DE 102010047074
(43) Veröffentlichungstag der Anmeldung: 07.08.2013
(73) Patentinhaber: Fraunhofer-Gesellschaft zur Förderung der angewandten Forschung e.V., 80686 München (DE); Rheinisch-Westfälisch-Technische Hochschule Aachen, 52056 Aachen (DE)
(72) Erfinder: REFLE, Oliver, 72768 Reutlingen (DE); BORCHERS, Kirsten, 70569 Stuttgart (DE); SEILER, Nadine, 52072 Aachen (DE); KRÜGER, Hartmut, 12163 Berlin (DE); JÄGER, Raimund, 79108 Freiburg (DE); GRAF, Careen, 71229 Leonberg (DE); KLUGER, Petra, 70839 Gerlingen (DE); RIESTER, Dominik, 52062 Aachen (DE); MEYER, Wolfdietrich, 14471 Potsdam (DE); BIERWISCH, Claas, 79108 Freiburg (DE); TOVAR, Günter, 71111 Waldenbuch (DE); ENGELHARDT, Sascha, 52069 Aachen (DE); BREMUS-KÖBBERLING, Elke, 41466 Neuss (DE); WEGENER, Michael, 16816 Neuruppin (DE)
(74) Vertreter: Rösler, Uwe
(86) Internationale Anmeldenummer: PCT/EP2011/004931
(87) Internationale Veröffentlichungsnummer: WO 2012/041522

(56) Entgegenhaltungen:
- WO-A1-01/26023
- WO-A2-2007/106497

## Beschreibung

### Technisches Gebiet

Die Erfindung bezieht sich auf eine Vorrichtung sowie ein Verfahren zur schichtweisen Herstellung von 3D-Strukturen mit einer Druckkopfanordnung, die relativ zu einer Arbeitsebene kontrolliert positionierbar ist und mit wenigstens zwei Reservoirbehältern verbunden ist, in denen flüssig bis pastöses photovernetzbares Material mit jeweils unterschiedlichen Photoempfindlichkeiten bevorratet ist, das jeweils über die Druckkopfanordnung in den Bereich der Arbeitsebene ortsselektiv ausbringbar ist, sowie mit einer Strahlungsquellenanordnung, die elektromagnetische Strahlung in Abhängigkeit der Photoempfindlichkeit des ortsselektiv auf die Arbeitsebene ausgebrachten photovernetzbaren Materials flächig emittiert.

### Stand der Technik

Die Herstellung großvolumiger Teile von mehreren Kubikzentimetern Größe, die zudem über sehr feine Strukturierungen oder Strukturunterbereiche im Mikro- und Submikrometerbereich verfügen, stellt eine verfahrenstechnische Herausforderung dar. Sollen derartige Teile überdies möglichst in einstückiger Bauform und mit einer hohen Formfreiheit hergestellt werden können, so bieten sich für ein derartiges technisches Vorhaben bevorzugt oder letztlich ausschließlich Techniken auf dem Gebiet generativer Herstellverfahren an.

In diesem Zusammenhang ist ein laserbasiertes Aufbauverfahren gemäß dem Stereolithographie-Verfahren, kurz SL-Verfahren zu nennen, bei dem ein in einer Arbeitsebene flächig als Schicht aufgetragener, lichtaushärtender Kunststoff von einem Laser ortsselektive ausgehärtet wird. Die Prozedur erfolgt in einem Bad, welches mit einem flüssigen oder pastösen Basismaterial des lichtempfindlichen Kunststoffes gefüllt ist. Die sich ortsselektiv durch Laserlicht induzierte Materialverfestigung ausbildenden Strukturbereiche in der Arbeitsebene werden in einem nächsten Schritt um den Betrag einer Schichtstärke nach unten in das Bad verfahren, so dass sich erneut eine Kunststoffschicht über den verfestigten Strukturbereichen innerhalb der Arbeitsebene ausbilden kann. Eine Vergleichmäßigung der sich erneut ausbildenden Kunststoffschicht erfolgt üblicherweise mit einer Wischereinheit. Unter Zugrundelegung von CAD-Daten, die die Form der herzustellenden Struktur beschreiben, wird der Laserstrahl kontrolliert durch bewegliche Spiegel längs der Arbeitsebene bewegt, so dass sich die belichteten Kunststoffschichtbereiche verfestigen und sich mit den bereits in einer Schichtlage unteren bereits verfestigen Strukturen einstückig verbinden. Diese Prozessabfolge wird schritt- bzw. schichtweise solange wiederholt bis sich nach und nach eine dreidimensionale Struktur entsteht. Beispielsweise ist aus der DE 100 24 618 A1 ein derartiges stereolithographisches Verfahren zur Erzeugung dreidimensionaler Gegenstände bekannt, die durch Bestrahlen flüssiger bis gelartiger Silikonkautschuke mit IR-Laser aufgebaut werden.

SL-Verfahren haben aber den Nachteil, dass nur ein einziges Material für den Aufbau einer dreidimensionalen Struktur verwendet werden kann. Hinzukommt, dass die Strukturauflösung, d.h. die Strukturgrößendimensionierung, begrenzt ist, so dass Mikro- und vor allem Submikrometerstrukturen auf diese Weise nicht herstellbar sind. Auch sind Grenzen hinsichtlich der elastischen Struktureigenschaften gesetzt, zumal die mit dem SL-Verfahren verarbeitbaren Kunststoffmaterialien über formstabile und damit gering elastische Eigenschaften verfügen.

Eine weitere Verfahrensvariante für die Herstellung einstückiger Strukturen oder Bauteile mittels generativer Herstellungsverfahren stellt die so genannte 3D-Drucktechnik dar, die ein Fertigen dreidimensionaler Teile mit beinahe unbegrenzter Geometriefreiheit erlaubt und dies unter Verwendung mehrerer unterschiedlicher, Materialien, so dass beispielsweise Elastizitäten ortselektiv eingestellt werden können. Durch die einstückige Herstellung der Strukturen, kann auf ein späteres Fügen von Einzelteilen zur Herstellung komplexer Strukturen vollständig verzichtet werden. Somit löst dieses Verfahren bereits einen Großteil der mit dem SL-Verfahren verbundnen Probleme.

In der US 6 658,314 B1 ist ein vorstehend erwähntes 3D-Druckverfahren beschrieben, das eine Druckkopfanordnung nutzt, die relativ zu einer Arbeitsebene kontrolliert verfahrbar und positionierbar ist und mit wenigstens zwei Reservoirbehältern verbunden ist, in denen flüssig bis pastöses photovernetzbares Material mit jeweils unterschiedlichen Photoempfindlichkeiten bevorratet ist. Je nach Druckvorgabe, die letztlich durch einen CAD-Datensatz bestimmt ist, der die räumliche Ausbildung der herzustellenden Struktur beschreibt, wird ein geeignet gewähltes photovernetzbares Material über die Druckkopfanordnung in den Bereich der Arbeitsebene ortsselektiv ausgebracht. Typischerweise erfolgt ein Zweikomponentenaustrag aus der Druckkopfanordnung in die Arbeitsebene dergestalt, dass das photovernetzbare Material, das zur Ausbildung der Struktur dient, das so genannte Strukturmaterial, von einem Stützmaterial zumindest teilweise umgeben oder unterfüttert wird, das nicht notwendiger Weise aus einem photovernetzbaren Material bestehen muss. Im Anschluss wird mit einer Strahlungsquellenanordnung, die elektromagnetische Strahlung in Abhängigkeit der Photoempfindlichkeit des ortsselektiv auf die Arbeitsebene ausgebrachten photovernetzbaren Materials flächig emittiert, eine lichtinduzierte Verfestigung des Strukturmaterials. Der Vorgang des ortsselektiven Materialaustrages in der Arbeitsebene, der einem herkömmlichen Druckvorgang ähnelt, sowie der nachfolgende ganzflächige Beleuchtungsvorgang der Arbeitsebene wird vielfach wiederholt, wobei bereits verfestigte Strukturen gegenüber der Arbeitsebene abgesenkt werden, sodass die beiden Prozessschritte stets zur Neubildung von sich verfestigenden Strukturen in der Arbeitsebene dienen, wodurch eine dreidimensionale Struktur durch schicht- bzw. lagenweises Anwachsen entsteht.

Das bekannte Druckverfahren ist jedoch in der möglichen Strukturauflösung begrenzt und vermag keineswegs räumliche Strukturen mit Mikro- oder Submikrometer großen Unterstrukturen herzustellen. Unter Ausnutzung modernster Druckköpfe und mit bestens auf den Druck-Prozess abgestimmten Materialien sind derzeit Auflösungen von ca. 10 µm erreichbar.

Um Strukturen mit Strukturdimensionen von weniger als 10 µm im Rahmen generativer Herstellungsverfahren zur realisieren wird die so genannte Multiphotonenpolymerisation angewandt. Dieses Verfahren funktioniert ähnlich wie die Stereolithographie auf Basis der Vernetzung von Präpolymeren durch Bestrahlung mit einem Laserstrahl. Wie beim SL-Verfahren wird auch bei der Multiphotonenpolymerisation, kurz MPP, innerhalb eines Bades aus flüssigem photovernetzbarem Kunststoffmaterial in einer Arbeitsebene eine flächige photovernetzbare Materialschicht mit einer Wischereinheit hergestellt, die mit einem hochintensiven, fokussierten Laserstrahl ortsselektiv unter Vorgabe eines festgelegten Bestrahlungsmusters beleuchtet wird. Insbesondere dienen zur Zwei- oder Mehrphotonenanregung Kurzpulslaser, die in der Lage sind Pico- bis Femtosekundenpulse zu erzeugen, um auf diese Weise einen hohen Energieeintrag in ein kleines Volumen von wenigen Femtolitern in kurzer Zeit zu konzentrieren, in dem das Kunststoffmaterial verfestigende Multiphotonenprozesse ausgesetzt wird. Ein derartiges Verfahren ist bspw. in der DE 10152 878 B4 beschrieben.

Das MPP-Verfahren besitzt jedoch drei Nachteile:
(a) Es können zur Generierung von 3D-Strukturen typischerweise nur kleine Prozessgeschwindigkeiten benutzt werden, wodurch ein Aufbau von makroskopischen Bauteilen in einer annehmbaren Zeit nicht möglich ist.
(b) Wie beschrieben nutzt das MPP-Verfahren ein Polymerbad. Strukturen bestehend aus mehreren Materialien sind mittels MPP-Verfahren schwer bis gar nicht zu realisieren.
(c) Aufgrund der notwendigen starken Fokussierung und der im Polymer bestehenden Absorption und Streuung ist die Eindringtiefe in das Polymerbad auf Größen von ca. kleiner 1 mm begrenzt.

Ein weiteres Beispiel für die Herstellung dreidimensionaler Strukturen mittels generativer Herstellungstechnik ist in der Druckschrift WO 2007/106497 A2 beschrieben, mit der eine perforierte Hohlkörperstruktur im Wege eines tröpfchenweisen Abscheidens von mittels Multiphotonenprozesses polymerisierenden Kunststoffes erzeugbar ist.

### Darstellung der Erfindung

Der Erfindung liegt die Aufgabe zugrunde eine Vorrichtung sowie ein Verfahren zur schichtweisen Herstellung von 3D-Strukturen, die sowohl über makroskopische Dimensionen von wenigstens einigen Kubikzentimeter Größe sowie auch Strukturdimensionen im Mikro- und/oder Submikrometerbereich aufweisen, d.h. insbesondere Strukturdimensionen von kleiner 10 µm, anzugeben. Die 3D-Strukturen sollen in für industrielle Maßstäbe vertretbaren Produktionszeiten realisierbar sein. Ferner soll es möglich sein die 3D-Strukturen mit unterschiedlichen Materialien und Materialeigenschaften in einstückiger Bauweise herzustellen. Die hierzu zu treffenden Maßnahmen sollen die Verwendung von biokompatiblen Werkstoffen zur Herstellung der 3D-Strukturen nicht ausschließen, so dass die Herstellung biologischer Strukturen grundsätzlich möglich sein soll.

Eine lösungsgemäße Vorrichtung ist Gegenstand der Anspruches 1. Lösungsgemäßes Verfahren ist in Anspruch 16 beschrieben. Lösungsgemäße Verwendungen der Vorrichtung zur Herstellung bestimmter 3D-Strukturen ist Gegenstand der Ansprüche 11 bis 15. Sämtliche lösungsgemäßen Gegenstände werden durch die Merkmale der Unteransprüche sowie durch weiterführende Erläuterungen in vorteilhafter Weise weitergebildet.

Die lösungsgemäße Vorrichtung geht von einer an sich bekannten Vorrichtung zur schichtweisen Herstellung von 3D-Strukturen aus, die zur Durchführung der eingangs erläuterten 3D-Drucktechnik ausgebildet ist. So ist eine Druckkopfanordnung vorgesehen, die relativ zu einer Arbeitsebene kontrolliert positionierbar ist und mit wenigstens zwei Reservoirbehältern verbunden ist, in denen flüssig bis pastöses photovernetzbares Material mit jeweils unterschiedlichen Photoempfindlichkeiten bevorratet ist. Über die Druckkopfanordnung ist das jeweilige photovernetzbare Material in den Bereich der Arbeitsebene ortsselektiv ausbringbar. Mit Hilfe einer Strahlungsquellenanordnung, die elektromagnetische Strahlung in Abhängigkeit der Photoempfindlichkeit des ortsselektiv auf die Arbeitsebene ausgebrachten photovernetzbaren Materials flächig emittiert, wird schließlich das ortsselektiv ausgebrachte Material verfestigt. Lösungsgemäß umfasst die Strahlungsquellenanordnung wenigstens eine Laserlichtquelle, deren Laserstrahl mithilfe optischer Strahlablenk- und Fokussiermittel in einen Bereich einer auf die Arbeitsebene mittels der Druckkopfanordnung flächig ausbringbaren photovernetzbaren Materialschicht fokussierbar ist und im Fokusbereich innerhalb der photovernetzbaren Materialschicht Zweiphotonen- oder Mehrphotonenprozesse, die zur ortsselektiven Verfestigung des photovernetzbaren Materials führen, initiiert.

Die lösungsgemäße Vorrichtung vereint somit Vorteile und vermeidet die Nachteile, die jeweils mit der bekannten 3D-Drucktechnik sowie dem MPP-Verfahren verbunden sind. Ferner überwindet die lösungsgemäße Vorrichtung die Unterschiedlichkeit beider einzelnen Verfahrensvarianten. So werden bei dem herkömmlichen 3D-Druckverfahren die Materialien ortsselektiv aufgetragen, danach erfolgt eine flächige Bestrahlung der Arbeitsebene mit den darauf ortsselektiv aufgebrachten Strukturen. Demgegenüber geht das herkömmliche MPP-Verfahren von einem vollfächigen Bad aus flüssigem photovernetzbaren Material auf der Arbeitsebene aus, wohingegen die Verfestigung des photovernetzbaren Materials durch ortsselektive Bestrahlung erfolgt.

In der lösungsgemäßen Vorrichtung vermag die Druckkopfanordnung neben einem ortsselektiven Materialaustrag durch entsprechende an der Druckkopfanordnung vorgesehene einzelne Druckkopfdüsen auch einen Austrag von fotovernetzbarem Material zu realisieren, das unter Ausbildung einer flächigen Schicht mit einer einheitlichen Schichtdicke und einer planen Schichtoberfläche auf die Arbeitsebene ausbringbar ist.

In einer bevorzugten Ausführungsform weist die Druckkopfanordnung wenigstens zwei, insbesondere eine Vielzahl, insbesondere 50 bis 200 Druckdüsen auf.

In einer bevorzugten Ausführungsform der Druckkopfanordnung sieht diese eine Vielzahl längs einer Linie angeordnete Druckdüsen vor, durch die das jeweils fotovernetzbare Material gleichmäßig verteilt ausbringbar ist. Während des Materialaustrages wird die Druckkopfanordnung vorzugsweise orthogonal zur linienhaften Anordnung der einzelnen Druckdüsen relativ zur Arbeitsebene bewegt. Mit Hilfe einer derart ausgebildeten Druckkopfanordnung ist es möglich, auf die Bevorratung eines im flüssigen Zustand befindlichen fotovernetzbaren Materials innerhalb eines Bades, wie dies für die herkömmliche Ausführung von MPPbasierten mikrostrukturierten Polymerisationsprozessen der Fall ist, zu verzichten und stattdessen innerhalb der mittels 3D-Drucktechnik bzw. Inkjetdruck makroskopisch ortselektiv ausgebrachten gleichmäßigen, flächigen Schichten aus flüssigem fotovernetzbarem Material mittels MPP mikroskopische zwei- oder dreidimensionale Sub-Strukturen zu verfestigen. Durch die lösungsgemäße Ausbildung der Druckkopfanordnung und Strahlungsquellenanordnung ist die konstruktive Voraussetzung geschaffen, die an sich bekannte 3D-Drucktechnik mit der MPP-Verfahrensvariante innerhalb einer einzigen Vorrichtung zu kombinieren. Die Vorteile der lösungsgemäßen Vorrichtung kommen besonders dann zum Tragen, wenn makroskopische Objekte mit einem Volumen von wenigstens einigen Kubikzentimetern hergestellt werden, die sowohl Makrostrukturen als auch mikrostrukturierte Bereiche enthalten.

Makrostrukturen können mit den für 3D Inkjet Druckverfahren üblichen, im Rapid Prototyping genutzten fertigungs-tauglichen Geschwindigkeiten aufgebaut werden. Damit werden typischerweise Strukturauflösungen von etwa 100 µm, unter optimalen Bedingungen eine minimale Auflösung von etwa 10 µm erreicht. Bereiche, in denen kleinere Strukturen insbesondere im Bereich 0,5 µm bis 200µm, bevorzugt 0,5 µm bis 10 µm, bevorzugt 0,1 µm bis 200 µm, bevorzugt 0,1 µm bis 200 µm, bevorzugt 0,5 µm bis 50 µm, bevorzugt 1 µm bis 200 µm, bevorzugt 0,1 µm bis 50 µm, bevorzugt 1 µm bis 50 µm erzeugt werden sollen, werden vorzugsweise nach dem hochauflösenden MPP Verfahren verfestigt. Dabei werden Strukturen in zuvor durch den Druckkopf abgesetzte Materialschichten eingeschrieben. Selbstverständlich kann das MPP-Verfahren auch für die Herstellung von Strukturen mit Strukturdimensionen im Bereich von größer 200 µm eingesetzt werden, so dass eine Überlappung beider Verfahrenstechniken zur Herstellung von Strukturen im Übergangsbereich zwischen Mikro- und Makrostrukturen genutzt werden kann. Durch die nahtlose Kombination der mikrostrukturierenden und der makrostrukturierende Fertigungstechnik werden für makroskopische Objekte mit mikrostrukturierten Bereichen Produzierzeiten erreicht, die für industrielle Maßstäbe vertretbar sind.

Darüber hinaus ist in vorteilhafter Weise dafür Vorsorge getroffen, dass beide innerhalb der Vorrichtung kombiniert anzuwendenden Verfahrenstechniken, d.h. 3D-Drucktechnik sowie MPP-Verfahrenstechnik keine störende Fotoquerempfindlichkeiten aufweisen, d.h. die ganzflächige Bestrahlung der Arbeitsebene mit dem darauf ortsselektiv aufgebrachten fotovernetzbarem Material im Wege der Drucktechnik vermag eine bspw. bereits in einem vorangegangenen Prozessschritt flächig auf der Arbeitsebene unter Anwendung des MPP-Verfahrens aufgebrachte fotovernetzbare Materialschicht nicht zu beeinträchtigen. Hierzu werden für die jeweils unterschiedlichen Verfahrensweisen jeweils fotovernetzbares Material gewählt, dessen Fotoempfindlichkeiten sich deutlich voneinander unterscheiden.

Somit ist es erforderlich innerhalb der Reservoirbehälter jeweils fotovernetzbares Material zu bevorraten, das über optisch unterschiedliche Absorptionseigenschaften verfügt. Die Absorptionseigenschaften von fotovernetzbaren Materialien werden durch den Zusatz wellenlängenselektiver Fotoinitiatoren bestimmt. Die innerhalb der fotovernetzbaren Materialien eingebrachten Fotoinitiatoren vermögen elektromagnetische Strahlung geeigneter Wellenlänge zu absorbieren, wodurch innerhalb des fotovernetzbaren Materials Materialverfestigungen ausgelöst werden.

Für die kombinierte Ausführung der 3D-Drucktechnik und des MPP-Verfahrens in jeweils getrennten Prozessschritten weist eine bevorzugte Ausführungsform der Strahlungsquellenanordnung zur ganzflächigen Beaufschlagung der Arbeitsebene mit elektromagnetischer Strahlung eine Licht emittierende Diode bzw. Diodenanordnung auf, die ein erstes Wellenlängenspektrum emittiert, in dem zugleich der Absorptionsbereich des ersten fotovernetzbaren Materials liegt, das im Wege der 3D-Drucktechnik auf die Arbeitsebene ausgebracht wird. Demgegenüber emittiert die Laserlichtquelle Laserstrahlung mit einer Wellenlänge, die sich vom ersten Wellenlängenspektrum unterscheidet, und die von einem zweiten durch das Inkjetverfahren aufgebrachten Material absorbiert wird, welches dadurch mittels MPP in einer Substruktur verfestigt wird.

Die physikalische Natur einer Multiphotonenanregung innerhalb des fotovernetzbaren Materials, das im Rahmen des MPP-Verfahrens auf die Arbeitsebene ausgebracht wird, ermöglicht grundsätzlich auch die Ausbildung einer Ausführungsvariante mit einer Strahlungsquellenanordnung, die eine Laserlichtquelle als einzige Strahlungsquelle umfasst. So treten Zwei- oder Mehrphotonenabsorptionsprozesse nur unter bestimmten Bedingungen auf. Um diese Bedingungen zu erreichen wird fotoempfindliches Material einer sehr hohen kurzzeitig wirkenden Bestrahlungsintensität ausgesetzt, wie dies beim Einsatz von fokussierten Piko- oder Femtosekundenkurzzeitlaserpulsen der Fall ist. Durch optisch nicht lineare Prozesse, die mit einer Frequenzverdopplung bzw. Wellenlängenhalbierung vergleichbar sind, können Multiphotonenanregungen innerhalb des photovernetzbaren Materials im Fokusbereich initiiert werden, die durch Polymerisationsreaktionen das Material lokal verfestigen. Alle übrigen Materialbereiche, in denen die vorstehend erläuterten optischen Bedingungen nicht gegeben sind, stellen für die Laserstrahlung transparente Materialbereiche dar.

Wählt man demgegenüber unter Nutzung der 3D-Drucktechnik fotovernetzbares Material mit auf die Laserwellenlänge abgestimmten Photoinitiatoren, so kann das ortsselektiv auf die Arbeitsebene deponierte fotovernetzbare Material durch Wechselwirkung mit dem Laserlicht verfestigt werden.

Im Unterschied zur vorstehend erhobenen Forderung der Verwendung wenigstens zweier Lichtquellen mit unterschiedlichen Emissionsspektren bedarf es unter gezielter Nutzung des optisch nicht linearen Multiphotonenprozesses lediglich einer einzigen Lichtquelle, nämlich eines Lasers, dessen Laserstrahlung durch entsprechende Wahl der am Ort des zu bestrahlenden photovernetzbaren Materials zu deponierenden Lichtintensität eine unterschiedliche Wellenlängencharakteristik erfährt. Wird der Laserstrahl unfokussiert oder mit aufgeweiteten Strahlquerschnitt auf die Materialoberfläche gerichtet, d.h. mit normaler oder geringer Lichtintensität, so treten Lichtabsorptionen bei geeigneter Materialwahl bei der Laserwellenlänge auf. Wird hingegen der Laserstrahl fokussiert und auf diese Weise die deponierte Lichtintensität stark erhöht, so treten in einem geeignet gewählten Material Zwei- oder Mehrphotoneneffekte auf, die einer Lichtabsorption mit Licht der halben Laserwellenlänge entsprechen. Selbstverständlich bedarf es auch in diesem Fall einer geeigneten Auswahl von fotovernetzbaren Materialien in Hinblick auf ihre Photoempfindlichkeiten, so dass das mittels der 3D-Drucktechnik auszubringende Material entweder das Laserlicht mit reduzierter Strahlintensität absorbiert, oder im Wege der Vernetzung mittels des MPP-Verfahrens ausschließlich mit dem fokussierten, hochintensiven Kurzzeitlaserpuls wechselwirkt.

In besonders vorteilhafter Weise vermag die Druckkopfanordnung über die Vielzahl der linear angeordneten Druckdüsen das fotovernetzbare Material unter Ausbildung einer möglichst homogenen Materialschicht hinsichtlich Materialschichtdicke sowie auch hinsichtlich einer möglichst ebenen bzw. planar ausgebildeten Schichtoberfläche auszutragen. Für eine zuverlässige und hoch qualitative Ausbildung von Mikro- und Submikrometer große Strukturen innerhalb der flächig ausgebrachten Materialschicht mit Hilfe des fokussierten Laserstrahls bedarf es jedoch einer möglichst glatten Materialschichtoberfläche sowie eine Materialschicht mit möglichst homogener Materialschichtdicke. Da die jeweils in die Arbeitsebene aufgetragenen Materialschichten sehr dünn sind, besteht eine fertige 3D-Struktur von nur einigen Millimetern Bauhöhe typischerweise aus mehreren Hundert Einzelschichten. Dementsprechend würden kleinste Unterschiede in den jeweils aufgetragenen Mäterialschichtmengen zu erheblichen Abweichungen in der Bauteilgeometrie führen. Bei der konventionell eingesetzten 3D-Drucktechnik wird dieses Problem dadurch gelöst, dass etwas mehr Material auf der Arbeitsebene aufgebracht wird, als für die tatsächliche Materialschichtdicke eigentlich notwendig wäre. Anschließend wird durch eine mechanische Nivellier-Vorrichtung, beispielsweise in Form einer Rolle oder eines Schiebers das aufgetragene Material eingeebnet und überschüssiges Material abgetragen, um die Schicht auf eine exakte Nennhöhe zu bringen. Im Fall der lösungsgemäßen Kombination der MPP-Technik und der 3D-Drucktechnik würde eine derartige mechanische Glättung zu einer mechanischen Krafteinwirkung und damit verbunden einer Deformation oder gar Zerstörung bereits feinster, im Wege des MPP-Verfahrens erzeugter Strukturen führen.

In einer bevorzugten Ausführungsform ist daher ein berührungslos arbeitendes Messsystem vorgesehen, das die Schichtdicke und/oder die Schichtoberflächenbeschaffenheit der auf der Arbeitsebene abgeschiedenen photovernetzbaren Materialschicht erfasst, beispielsweise mittels optischer Messtechnik. Mit Hilfe einer Regeleinheit, die die seitens des Messsystems generierten Messsignale im Wege eines Soll-Ist-Vergleiches mit Referenzdaten vergleicht, wird die Druckkopfanordnung im Falle fehlerhaft festgestellter Schichtdicken und/oder festgestellter Schichtoberflächenbeschaffenheiten zu Nachkorrekturmaßnahmen entsprechend angesteuert.

In einer weiteren bevorzugten Ausführungsform ist eine durch die Regeleinheit ansteuerbare Wärmequelle vorgesehen, die die auf der Arbeitsebene abgeschiedene photovernetzbare Materialschicht kontrolliert zu wärmen vermag, um auf diese Weise eine verbesserte Vergleichmäßigung insbesondere der Schichtoberflächenbeschaffenheit zu bewirken.

Die lösungsgemäße Vorrichtung eignet sich in besonders vorteilhafter Weise zur Herstellung makroskopischer Strukturen, die typischerweise einen Bauraum von mehreren Kubikzentimetern aufweisen können und die zumindest bereichsweise mikro- oder submikrometer große Unterstrukturen enthalten. Um derartig in ihren Strukturdimensionen hybrid zusammengesetzte Strukturen in einer für industrielle Maßstäbe vertretbaren Verfahrenszeit herstellen zu können, weist die Vorrichtung wenigstens drei Reservoireinheiten auf, die jeweils mit der Druckkopfanordnung verbunden sind. In einer ersten Reservoireinheit ist Stützmaterial enthalten, das selbst nicht notwendigerweise photovernetzbares Material darstellen muss. In wenigstens einer zweiten Reservoireinheit ist ein photovernetzbares Material enthalten, das zum Aufbau der Struktur im Wege eines ortsselektiven Materialaustrages gemeinsam mit dem Stützmaterial auf der Arbeitsebene deponiert wird. Im Weiteren soll dieses photovernetzbare Material als Strukturmaterial bezeichnet werden. Schließlich ist in wenigstens einer dritten Reservoireinheit photovernetzbares Material vorgesehen, das im Wege von laserstrahlinduzierten Zweiphotonen- oder Mehrphotonenprozessen innerhalb der mittels Inkjetdruck ortsselektiv aufgetragenen Materialschicht in einer dreidimensionalen Mikro-Substruktur verfestigbar ist.

Um die makroskopischen Strukturbereiche einer 3D-Struktur auszuformen, d. h. jene Bereiche, deren Strukturgrößen typischerweise größer als 100 µm messen, bedient man sich der 3D-Drucktechnik in Kombination mit flächiger Verfestigung. Das bedeutet, dass in die Arbeitsebene jeweils ortsselektiv das Stütz- sowie auch das photovernetzbare Strukturmaterial über wenigstens zwei unterschiedliche Druckdüsen der Druckkopfanordnung ortsselektiv auf jeweils eine gemeinsame Arbeitsebene ausbringbar sind. Im Anschluss daran erfolgt eine großflächige Belichtung des ortsselektiv ausgebrachten Materials, die das Strukturmaterial polymerisiert und dadurch verfestigt. Diese Prozessabfolge wird schicht- bzw. lagenweise vielfach wiederholt um die makroskopischen Strukturbereiche aufzubauen. Gilt es hingegen Strukturen im Mikro- und insbesondere Submikrometerbereich auszubilden, die mit den makroskopischen Strukturen einstückig zu verbinden sind, so bedarf es des Ausbringens des in wenigstens der dritten Reservoireinheit bevorrateten photovernetzbaren Materials über den Querschnitt der zuvor aufgebauten Struktur auf der Arbeitsebene unter Ausbildung einer photovernetzbaren Materialschicht, die nachfolgend ortsselektiv mit einer fokussierten elektromagnetischen Strahlung, vorzugsweise einem Laserstrahl derart bestrahlt wird, sodass im Fokusbereich innerhalb der photovernetzbaren Materialschicht Zweiphotonen- oder Mehrphotonenprozesse, die zur ortsselektiven Verfestigung im Mikrometer- und Submikrometerbereich des jeweils zweiten photovernetzbaren Materials führen, initiiert werden. Derartige Strukturen im unteren Mikro- und insbesondere Submikrometerbereich können Strukturgrößen im Bereich zwischen 0,1 µm bis zu 100 µm, bzw. typischerweise zwischen 0,4 µm bis zu 100 µm aufweisen. Die erzielbaren kleinsten Strukturdimensionen hängen von der Wahl der Wellenlänge der jeweils eingesetzten elektromagnetischen Strahlung sowie der Wellenlänge der an den Zweiphotonen- oder Mehrphotonenprozesse beteiligten Photonen ab. Derzeit technisch verfügbare Lasersysteme vermögen Laserlicht mit kleinsten Wellenlängen zwischen 0,15 µm und 0,2 µm zu erzeugen. Denkbar sind jedoch auch technologische Weiterentwicklungen derartiger Lasersysteme, insbesondere unter Nutzung optisch nichtlinearer Prozesse bzw. Frequenzvervielfachung, sodass Strukturgrößen im unteren Nanometerbereich, d.h. 10 nm bis 100 nm, grundsätzlich nicht auszuschließen sind.

Sowohl die Herstellung makroskopischer sowie auch mikroskopischer oder submikroskopischer Strukturbereiche erfolgt in einstückig zusammenhängender mit Prozessgeschwindigkeiten, die wenigstens annähernd typisch sind für die an sich bekannte 3D-Drucktechnik. Auf diese Weise lassen sich somit großvolumige Körper, die über feine Strukturierungen verfügen ökonomisch schnell und einstückig aufbauen. Außerdem ist es möglich, durch das 3D Druckverfahren unter Verwendung weiterer Materialsreservoireinheiten mehrere, unterschiedliche Struktur-Materialien miteinander zu kombinieren, die bspw. über unterschiedliche elastische Eigenschaften verfügen. Bspw. lassen sich unterschiedliche photovernetzbare Materialien in aufeinander folgenden Schichten oder makroskopisch ortselektiv innerhalb einer Arbeitsebene abscheiden und flächig oder mikrostrukturiert verfestigen, wodurch individuelle Struktureigenschaften erzeugt werden könne. Die so hergestellten Strukturen können gezielt variiert und reproduziert werden.

Ferner ist es denkbar durch die Verwendung mehrerer unterschiedlicher photovernetzbarer Materialien auch biofunktionale Substanzen in das Bauteil bzw. die großvolumige Struktur einzubringen, um auf diese Weise vor dem Hintergrund biologischer Anwendungen bestimmte Biofunktionalisierungen zu erreichen.

Mit Hilfe der lösungsgemäß ausgebildeten Vorrichtung und der damit realisierbaren neuartigen Verfahrensvariante, die eine Kombination die 3D-Drucktechnik und MPP-Verfahren ermöglicht, können bspw. aus dem biologischen Gewebebereich stammende Gefäßstrukturen nachgebildet werden und zum Transport von Körperflüssigkeiten oder Nährmedien oder weitere Flüssigkeiten eingesetzt werden. So ist es möglich, makroskopisch ausgebildete Röhren oder Schläuche, insbesondere verästelte einstückige Röhrensystemen herzustellen, deren Röhrendurchmesser bevorzugt bereichsweise von 5 mm bis 0,5 µm, insbesondere 5 mm bis 0,1 µm variieren, wobei vorzugsweise das Röhrensystem von Anschluss zu Anschluss eine Gesamtlänge von 0,5 cm bis 10 cm, vorzugsweise 0,5 cm bis 5 cm, insbesondere 1 cm bis 10 cm aufweist, und beispielsweise die Röhrenwände mit Poren zu versehen, deren Porendurchmesser im unteren Mikro- sowie Submikrometerbereich liegen, bevorzugt Strukturgrößen von maximal, bevorzugt kleiner 10 µm bis zu 0,1 µm.

Insbesondere für die erfindungsgemäße Herstellung von 3D-Strukturen, die zumindest teilweise aus biokompatiblen und/oder möglicherweise biologischen Materialien bestehen eignet sich als photovernetzbares Material in besonderer Weise ein Material, das die folgenden Komponenten aufweist:
i) mindestens eine polymere Vernetzer-Komponente mit mindestens zwei photovernetzbaren Gruppen, die aus der Gruppe ausgewählt sind, bestehend aus Acrylat, Methacrylat, Acrylamid, Methacrylamid, Urethanacrylat, Urethanmethacrylat, Ureaacrylat und Ureamethacrylat und ii) mindestens eine Photoinitiator-Komponente.

Zur Herstellung mindestens einer Schicht der dreidimensionalen Struktur, wird zunächst das photovernetzbare Material, umfassend mindestens eine polymere Vernetzer-Komponente und mindestens eine Photoinitiator-Komponente, auf die Arbeitsebene aufgebracht, und in einem nächsten Schritt das aufgebrachte photovernetzbare Material durch elektromagnetische Strahlung fixiert. Im Rahmen der Bestrahlung reagiert zumindest ein Teil, insbesondere im Wesentlichen alle, vorzugsweise alle der photovernetzbaren Gruppen des photovernetzbaren Materials miteinander, wodurch ein photovernetztes Material erhalten wird. Die Fixierung findet dergestalt statt, dass durch die elektromagnetische Strahlung die in dem photovernetzbaren Material vorhandene Photoinitiator-Komponente, insbesondere zur Spaltung, angeregt wird, um eine photo-initiierte Polymerisierungsreaktion der photovernetzbaren Gruppen des photovernetzbaren Materials zu starten. Durch diese kontrollierte Kettenreaktion wird zumindest ein Teil, bevorzugt im Wesentlichen alle, vorzugsweise alle der in den elektromagnetisch bestrahlten Bereichen liegenden photovernetzbaren Gruppen umgesetzt.

Die Abfolge der Verfahrensschritte bezüglich des Aufbringens mindestens eines photovernetzbaren Materials auf die Arbeitsebene, im weiteren als Schritt a) bezeichnet, und des Fixierens des mindestens einen aufgebrachten photovernetzbaren Materials durch elektromagnetische Strahlung, im weiteren als Schritt b) bezeichnet, führt zur Bildung einer Schicht des aufgebrachten Materials. Die erfindungsgemäß bevorzugte wiederholte Abfolge dieser Verfahrensschritte führt zur Bildung einer entsprechenden Zahl von Schichten.

In einer bevorzugten Ausführungsform wird das photovernetzbare Material durch die elektromagnetische Strahlung innerhalb einer aufgebrachten Schicht mittels Zwei- oder Multiphotonenprozessen in allen drei Raumrichtungen x, y und z, also dreidimensional, flächig oder ortsselektiv, fixiert. Bevorzugt entsteht durch eine Verfahrensfolge der Schritte a) und b) eine zwei- oder dreidimensionale Substruktur innerhalb einer Schicht.

Das Aufbringen des photovernetzbaren Materials erfolgt entweder flächig oder ortsselektiv, wobei das darauf folgende Fixieren des photovernetzbaren Materials zu einem photovernetzten Material durch eine elektromagnetische Strahlung erfolgt, die in bevorzugter Ausführungsform insbesondere auf die in dem photovernetzbaren Material enthaltenen Photoinitiator-Komponente abgestimmt ist und darüber hinaus eine ortsselektive oder flächige Bestrahlung gewährleistet. Mit Hilfe der Verfahrensfolge der Schritte a) und b) wird eine Schicht der zwei- oder dreidimensionalen Struktur aufgebaut. Es ist bevorzugt durch ein-, mehr- oder vielmalige Wiederholung der Verfahrensfolge a) und b) zwei, mehrere oder viele Schichten bereitzustellen. Bei jeder weiteren Verfahrensfolge der Schritte a) und b) erfolgt demgemäß eine Erstellung einer weiteren Schicht, die sich kovalent mit dem bereits photovernetzten Material verbindet.

Die eingesetzten photovernetzbaren Materialien sind in verschiedenen an sich bekannten Verfahren zur Herstellung einer zwei- oder dreidimensionalen Struktur, beispielsweise SL-Verfahren, 3D-Druck-Verfahren und MPP-Verfahren (MPP = Multiphotonenprozesse) in bevorzugter Ausführungsform universell einsetzbar, dass heißt ohne dass in bevorzugter Ausführungsform wesentliche weitere oder weitere Anpassungen für die einzelnen Verfahren notwendig sind. Die erfindungsgemäß hergestellte zwei- oder dreidimensionale Struktur weist vorzugsweise gewünschte Polymereigenschaften für beispielsweise Implantate auf.

Die eingesetzten photovernetzbaren Materialien zeichnen sich insbesondere durch eine geeignete Oberflächenspannung und Viskosität, insbesondere durch eine Viskosität von weniger als 200 mPa·s, insbesondere weniger als 80 mPa·s, besonders bevorzugt weniger als 40 mPa·s, aus. Diese Viskosität kann insbesondere durch Lösungsmittel, insbesondere durch einen Reaktivverdünner, mit einem Anteil von weniger als 51 % erreicht werden.

In einer-bevorzugten Ausführungsform beträgt die Oberflächenspannung des photovernetzbaren Materials weniger als 80 mN/m, insbesondere weniger als 70 mN/m, insbesondere weniger als 35 mN/m.

Des Weiteren weisen die photovernetzbaren Materialien bevorzugt die für die Verfahren zur Herstellung einer zwei- oder dreidimensionalen Struktur, insbesondere für SL-Verfahren, 3D-Druck-Verfahren und MPP-Verfahren, erforderliche, insbesondere hohe, Lichttransparenz für die Aushärtungswellenlänge der elektromagnetischen Strahlung und eine ausreichende Aushärtungsgeschwindigkeit auf. Die Lichttransparenz liegt dabei bevorzugt im VIS-NIR-Bereich oder UV-Bereich. Insbesondere wird die elektromagnetische Strahlung zusätzlich durch die photovernetzbaren Materialien absorbiert.

Das photovernetzbare Material verfügt hinsichtlich seiner Photovernetzbarkeit über ausreichende Vernetzungsgrade, ist selektiv durch die elektromagnetische Strahlung fixierbar und reagiert bevorzugt selektiv auf eine bevorzugt vorgesehene flächige und ortsaufgelöste Vernetzung. Des Weiteren genügt das photovernetzbare Material insbesondere den Anforderungen der 3D-Druckverfahren, beispielsweise InkJet-Drucken, hinsichtlich der einzuhaltenden Viskosität, des Verlaufsverhalten und der Druckstabilität.

Vorzugsweise wird als eine Komponente des photovernetzbaren Materials mindestens eine Photoinitiator-Komponente eingesetzt. Die Photoinitiator-Komponente ermöglicht eine möglichst effektive und selektive Fixierung des photovernetzbaren Materials, insbesondere in Kombination mit einer hinreichend schnellen Aushärtungsgeschwindigkeit des photovernetzbaren Materials. Die eingesetzte Photoinitiator-Komponente weist insbesondere einen hohen Photonabsorptionsquerschnitt, insbesondere einen hohen Zwei-Photonabsorptionsquerschnitt im VIS-NIR, und bevorzugt eine hohe Quantenausbeute auf.

In Zusammenhang mit der vorliegenden Erfindung wird unter dem Begriff "flächig", insbesondere einem flächigen Aufbringen eines photovernetzbaren Materials oder einem flächigen Fixieren eines aufgebrachten photovernetzbaren Materials verstanden, dass das Aufbringen des Materials oder die fixierende Strahlung gleichmäßig über die gesamte zu beschichtende oder fixierende Materialschicht erfolgt. Demgemäß kann ein flächiger Auftrag eines Materials oder ein flächiges Einwirken der Strahlung zur Ausbildung von dreidimensional ausgebildeten beziehungsweise fixierten Schichten führen. Insbesondere aufgrund des flächigen Materialauftrags beziehungsweise flächigen Einwirkens der Strahlung wird das photovernetzbare Material gleichmäßig aufgebracht beziehungsweise fixiert.

In Zusammenhang mit der vorliegenden Erfindung wird unter dem Begriff "ortsselektiv", insbesondere einem ortsselektiven Aufbringen eines photovernetzbaren Materials oder einem ortsselektiven Fixieren eines aufgebrachten photovernetzbaren Materials verstanden, dass das Aufbringen des Material oder die fixierende Strahlung nicht gleichmäßig über die gesamte zu beschichtende oder fixierende Materialschicht erfolgt.

Unter dem Begriff "polymere Vernetzer-Komponente mit mindestens zwei endständigen photovernetzbaren Gruppen" ist ein unverzweigtes oder mindestens einfach verzweigtes Polymer oder Oligomer zu verstehen, an dem die mindestens zwei photovernetzbaren Gruppen derart kovalent verbunden sind, dass sie zur Fixierung durch die elektromagnetische Strahlung in Schritt b) zur Verfügung stehen. Die polymere Vernetzer-Komponente weist funktionelle Gruppen auf, an denen die photovernetzbaren Gruppen kovalent gebunden sind. Bevorzugt findet diese kovalente Bindung zwischen der polymeren Vernetzer-Komponente und den photovernetzbaren Gruppen über eine Ester- oder Amidbindung statt. Als "polymere Vernetzer-Komponente" wird die Komponente bezeichnet, an der die photovernetzbaren Gruppen kovalent gebunden sind.

Unter dem Begriff "zweidimensionale Struktur" wird in einem dreidimensionalen Raum mit den Raumachsen x-y-z eine Struktur mit Kantenlängen x'-y'-z' entlang der Raumachsen verstanden, bei der die Länge der kürzesten Kante von x' und y' einer durch die Kanten x' und y' aufgespannten Fläche deutlich größer als die Kantenlänge z' ist, vorzugsweise um den Faktor 5, vorzugsweise 10, vorzugsweise 20, vorzugsweise 30, vorzugsweise 40, vorzugsweise 50, vorzugsweise 100, vorzugsweise 1000, bevorzugt 10000. Der Begriff "zweidimensionale Struktur" bedeutet demgemäß nicht, dass keine räumliche Ausdehnung in Richtung der dritten Dimension erfolgt. Bevorzugt weist die zweidimensionale Struktur in Richtung der dritten Dimension 1 bis 50 Schichten auf, insbesondere 1 bis 40 Schichten, bevorzugt 1 bis 20 Schichten, bevorzugt 1 bis 10 Schichten und besonders bevorzugt 5 bis 10 Schichten auf. Beispielsweise werden als zweidimensionale Strukturen Membranen, Vliese, hautähnliche Implantate und Netze verstanden.

Unter dem Begriff "kurzkettige Vernetzer-Komponente mit mindestens drei endständigen photovernetzbaren Gruppen" ist ein verzweigtes mehrfach funktionalisiertes Molekül zu verstehen, das bevorzugt eine maximale Kettenlänge pro Verzweigung von 10, bevorzugt 8, bevorzugt 6, aufweist. Die mindestens drei photovernetzbaren Gruppen sind derart mit diesem Molekül kovalent verbunden, dass sie zur Fixierung durch die elektromagnetische Strahlung in Schritt b) zur Verfügung stehen. Die polymere Vernetzer-Komponente weist funktionelle Gruppen auf, an denen die photovernetzbaren Gruppen kovalent gebunden sind. Bevorzugt findet diese kovalente Bindung zwischen der kurzkettigen Vernetzer-Komponente und den photovernetzbaren Gruppen über eine Ester- oder Amidbindung statt. Als "kurzkettige Vernetzer-Komponente" wird die Komponente bezeichnet, an der die photovernetzbaren Gruppen kovalent gebunden sind.

Unter dem Begriff "niedrig-viskose Modifikatorkomponente" wird eine Komponente verstanden, die bevorzugt eine Molmasse von weniger als 1000 g/mol und die Viskosität des photovernetzbaren Materials derart anpasst, so dass ein Viskositätsbereich gewährleistet ist, der zur universellen Anwendung der photovernetzbaren Materialien in den Verfahren zur Herstellung von zwei- oder dreidimensionalen Strukturen gewährleistet.

Bevorzugt weisen die niedrig-viskose Modifikatorkomponente und/oder die polymere und/oder kurzkettige Vernetzer-Komponente, weitere nicht-photovernetzbare funktionelle Gruppen auf, die nicht kovalent, insbesondere gar nicht, an die photovernetzbaren Gruppen binden können und damit für Kopplungsreaktionen, insbesondere mit biofunktionellen Komponenten, zur Verfügung stehen. Diese nicht-photovernetzbaren funktionellen Gruppen sind bevorzugt ausgewählt aus der Gruppe bestehend aus Hydroxy-, Cyanat-, Isocyanat-, Amino-, Imino-, Alken-, Alkin-, Carboxygruppe, bevorzugt Carboxygruppe.

Unter dem Begriff "Arbeitsebene" wird die Ebene verstanden, in der die in Schritt b) durchgeführte Fixierung durch die elektromagnetische Strahlung erfolgt. Vorzugsweise verläuft diese Ebene planar, im Wesentlichen planar, gewölbt oder im Wesentlichen gewölbt.

Unter dem Begriff "Verfahrensfolge der Schritte a) und b)" wird verstanden, dass zunächst das photovernetzbare Material in einem Schritt a) aufgebracht wird, entweder ortsselektiv oder flächig, und in einem Schritt b) das in Schritt a) aufgebrachte photovernetzbare Material flächig oder ortsselektiv fixiert wird, wobei vorzugsweise wenn Schritt a) ortsselektiv erfolgt, Schritt b) flächig oder wenn Schritt a) flächig erfolgt, Schritt b) ortsselektiv erfolgt.

Unter dem Begriff "photovernetzbare Materialien mit unterschiedlicher Photoempfindlichkeit" ist zu verstehen, dass die photovernetzbaren Materialien eine Photoinitiatorkomponente mit unterschiedlicher Photoempfindlichkeit aufweisen.

Die zwei- oder dreidimensionale Struktur wird als "biokompatibel" eingestuft, wenn nach 24 Stunden in einer zu untersuchenden Zellkultur mindestens 20%, bevorzugt mindestens 50%, bevorzugt mindestens 60%, bevorzugt mindestens 70%, bevorzugt mindestens 80% der Viabilität einer Vergleichszellkultur erreicht wird. Die Vergleichszellkultur weist die gleichen Zellen wie die zu untersuchenden Zellkultur auf. Lediglich hinsichtlich des Kultivierungsmediums unterscheiden sich beide Zellkulturen. Die zu untersuchende Zellkultur weist ein Kultivierungsmedium auf, das nach 24-stündiger Lagerung der zwei- oder dreidimensionalen Struktur in dem auch für die Vergleichszellkultur verwendeten Kultivierungsmedium erhalten wird. Die Viabilität (WST-Wert) wird bevorzugt mittels eines WST-1 Poliferationsassays bestimmt. Je nach Anwendung werden unterschiedliche Zelltypen verwendet, bevorzugt Endothelzellen, bevorzugt Chondrozyten.

Die in Schritt b) verwendete elektromagnetische Strahlung richtet sich in bevorzugter Ausführungsform nach den Anforderungen des in Schritt b) durchgeführten Fixierens. Die elektromagnetische Strahlung in Schritt b) muss die in dem photovernetzbaren Material verwendeten Photoinitiatoren selektiv anregen können, um so gezielt die Fixierung des photovernetzbaren Materials zu gewährleisten. Es ist bevorzugt die Strahlungsintensität der elektromagnetischen Strahlung in Abhängigkeit davon einzustellen, ob eine Fixierung flächig oder ortsselektiv, insbesondere über Zwei- oder Mehrphotonenprozesse, erfolgt. Es ist bevorzugt vorgesehen, die ortsselektive Fixierung mit Hilfe von Laserlicht, also lasergestützt, durchzuführen. Die flächige Fixierung findet bevorzugt mit Hilfe von UV-Licht statt, wobei bevorzugt der Spektralbereich an die Erfordernisse des durchgeführten Verfahrens oder an die Photoinitiator-Komponente angepasst wird. Insbesondere liegt der Spektralbereich bei 250 bis 500 nm. Als Quelle für das UV-Licht werden bevorzugt UV-Strahler, insbesondere mit eingeschränktem Spektralbereich, oder LED's (Leuchtdioden) verwendet.

In einer bevorzugten Ausführungsform weist die polymere Vernetzer-Komponente zwei, drei, vier, fünf oder mehr als 50, bevorzugt mehr als 70, bevorzugt mehr als 100, photovernetzbare Gruppen auf.

In einer bevorzugten Ausführungsform weist die polymere Vernetzer-Komponente zwei oder drei photovernetzbare Gruppen auf.

In einer bevorzugten Ausführungsform umfasst das photovernetzbare Material mindestens 2, mindestens 3, mindestens 4 oder mindestens 5 unterschiedliche polymere Vernetzer-Komponenten mit mindestens zwei photovernetzbaren Gruppen. In einer bevorzugten Ausführungsform weist die polymere Vernetzer-Komponente mit mindestens zwei photovernetzbaren Gruppen eine Molmasse von 300 bis 3000 g/mol auf.

In einer bevorzugten Ausführungsform ist die polymere Vernetzer-Komponente mit mindestens zwei photovernetzbaren Gruppen ein alpha,omega-Hydroxyoligomer, ein alpha,omega-Aminooligomer und/oder ein alpha-Hydroxy-omega-aminooligomer.

In einer bevorzugten Ausführungsform ist die polymere Vernetzer-Komponente aus der Gruppe ausgewählt bestehend aus Polyethylenglykol (PEG), Polypropylenglykol (PPG), Siloxane, Polytetrahydrofuran (PTHF), Bisphenol-A-ethoxylat (BPA-(EO)), Co-Block-Polyether davon, Biopolymere und modifizierte Biopolymere.

In einer bevorzugten Ausführungsform ist die polymere Vernetzer-Komponente ausgewählt aus der Gruppe bestehend aus Polyethylenglykol (PEG), Polypropylenglykol (PPG), Polytetrahydrofuran (PTHF), Bisphenol-A-ethoxylat (BPA-(EO)), Co-Block-Polyether davon, Biopolymere und modifizierte Biopolymere.

In einer bevorzugten Ausführungsform ist die polymere Vernetzer-Komponente mit mindestens zwei photovernetzbaren Gruppen ausgewählt aus der Gruppe bestehend aus PTHF(1400)-Diacrylat, PTHF(2000)-Diacrylat, PTHF(2900)-Diacrylat, PPG(2000)-Diacrylat, PPG(2300)-Diurethan-methacrylat und PTHF(1600)-Diurethanmethacrylat. Die in Klammern stehenden Zahlen geben das durchschnittliche Molekulargewicht der polymeren Vernetzer-Komponente an.

In einer bevorzugten Ausführungsform ist das Biopolymer, auch biologisches Makromolekül genannt, aus der Gruppe ausgewählt bestehend aus Proteine, Polysaccharide, Glucosaminglykane und Derivate davon.

In einer bevorzugten Ausführungsform ist das Protein ausgewählt aus der Gruppe bestehend aus Albumin, Kollagene, Gelatine und Fibronektin..

In einer bevorzugten Ausführungsform wird als photovernetzbares Material ein negative Ladungen aufweisendes modifiziertes Biopolymer, insbesondere Heparinsulfat, verwendet. Durch diese negativen Ladungen werden bevorzugt Wachstumsfaktoren, Analoga, Fragmente und/oder Derivate davon ionisch, insbesondere temporär, gebunden.

In einer bevorzugten Ausführungsform ist der Wachstumsfaktor ausgewählt aus der Gruppe bestehend aus VEGF (Vascular Endothelial Growth Factor), FGF (Fibroblast Growth Factor), PDGF (Platelet Derived Growth Factor), Pleitrophin, PIGF (Placenta Growth Factor), HGF/SF (Hepatocyte Growth Factor/ Scatter Factor) und Midkine.

In einer bevorzugten Ausführungsform ist das Polysaccharid ausgewählt aus der Gruppe bestehend aus Cellulose, Stärke und Glycogen.

In einer bevorzugten Ausführungsform ist das Glukosaminglykan ausgewählt aus der Gruppe bestehend aus Hyaluronsäure, Chondroitinsulfat, Dermatansulfat, Heparinsulfat und Heparin.

In einer besonderen Ausführungsform wird ein Teil der photovernetzbaren Gruppen in dem mindestens einen photovernetzbaren Material in Schritt b) nicht umgesetzt, insbesondere 1 bis 60 %, insbesondere 10 bis 50 %, insbesondere 20 bis 40 % der eingesetzten photovernetzbaren Gruppen.

Die nicht umgesetzten oder nicht fixierten photovernetzbaren Gruppen der photovernetzten Struktur stehen insbesondere zur weiteren Oberflächenfunktionalisierung und/oder Biofunktionalisierung zur Verfügung.

In einer bevorzugten Ausführungsform wird das photovernetzbare oder photovernetzte Material, z. B. auch ein als polymere Vernetzer-Komponente eingesetztes modifiziertes oder nicht modifiziertes Biopolymer, mit mindestens einer biofunktionellen Komponente funktionalisiert.

In einer bevorzugten Ausführungsform wird die mindestens eine biofunktionelle Komponente direkt oder indirekt mit dem photovernetzbaren oder photovernetzten Material verbunden.

Dementsprechend sieht eine bevorzugte Ausführungsform vor, dass das photovernetzbare Material vor dem Fixieren mit einer biofunktionellen Komponente, insbesondere vor dem Aufbringen, funktionalisiert wird. In einer weiteren bevorzugten Ausführungsform kann allerdings auch vorgesehen sein, dass die mindestens eine biofunktionelle Komponente nach dem Fixieren des photovernetzbaren Materials eingebracht wird, dass heißt an die Oberfläche des photovernetzten Materials kovalent oder nicht-kovalent gebunden wird.

In einer bevorzugten Ausführungsform werden die nicht umgesetzten, photovernetzbaren Gruppen mit mindestens einer biofunktionellen Komponente funktionalisiert.

In einer bevorzugten Ausführungsform werden die nicht-photovernetzbaren, funktionellen Gruppen der niedrig-viskosen Modifikatorkomponente und/oder kurzkettigen und/oder polymeren Vernetzer-Komponente mit mindestens einer biofunktionellen Komponente funktionalisiert. In einer bevorzugten Ausführungsform erfolgt diese Biofunktionalisierung über eine Amidbindung, wobei zur deren Bildung bevorzugt Carbodiimid als Reaktionsvermittler verwendet wird.

In einer bevorzugten Ausführungsform erfolgt die Biofunktionalisierung der zwei- oder dreidimensionalen Struktur durch gezielten Einbau biofunktioneller Komponenten in die zwei- oder dreidimensionale Struktur, insbesondere durch das Biofunktionalisieren des photovernetzbaren Materials vor dem Fixieren des photovernetzbaren Materials in Schritt b) oder an die nicht umgesetzten Doppelbindungen und/ oder an die nicht-photovernetzbaren funktionellen Gruppen des photovernetzten Materials. In beiden Ausführungsformen kann vorgesehen sein, dass das zu biofunktionalisierende Material, also das photovernetzte oder photovernetzbare Material, selbst ein Biopolymer oder ein modifiziertes Biopolymer ist bzw. umfasst. Die Biofunktionalisierung ist besonders bevorzugt bei der Verwendung der zwei- oder dreidimensionalen Struktur in biologischer oder medizinischer Verwendung, z. B. insbesondere als Aderersatzmaterial, z. B. um zelladhärente, proliferationsfördernde und/oder antithrombogene Eigenschaften auf die zwei- oder dreidimensionale Struktur zu übertragen. Die antithromogenen Eigenschaften der zwei- oder dreidimensionalen Struktur werden bevorzugt insbesondere über die sequenzielle Anbindung von modifiziertem Heparin, insbesondere Heparinsulfat, erreicht.

In einer bevorzugten Ausführungsform ist die biofunktionelle Komponente, die zur Biofunktionalisierung der photovernetzten Materials eingesetzt wird, ausgewählt aus der Gruppe bestehend aus Biopolymeren, modifizierten Biopolymeren, Proteinen wie Glycoproteinen, Wachstumsfaktoren oder Antikörper, Peptidsequenzen, Polysaccharide, Glykosaminglykane, Nucleinsäuren, Aptamere und Derivate davon.

In einer besonders bevorzugten Ausführungsform ist vorgesehen, dass die als biofunktionelle Komponente charakterisierten Biopolymere oder modifizierten Biopolymere auch als polymere Vernetzer-Komponenten des photovernetzbaren Materials eingesetzt werden können. In einer weiteren bevorzugten Ausführungsform ist vorgesehen, dass die als Biopolymere oder modifizierte Biopolymere aufgeführten polymeren Vernetzer-Komponenten des photovernetzbaren Materials auch als biofunktionelle Komponenten eingesetzt werden können zur Biofunktionalisierung des photovernetzten oder photovernetzbaren Materials, insbesondere der polymeren oder kurzkettigen Vernetzer-Komponente, insbesondere wenn die polymere Vernetzer-Komponente als Biopolymer oder modifiziertes Biopolymer ausgeführt ist.

In einer bevorzugten Ausführungsform wird in Schritt a) acrylierte Gelatine als polymere Vernetzer-Komponente mit mindestens zwei photovernetzbaren Gruppen verwendet. Die nach Schritt b) photovernetzte Struktur enthält dadurch Anknüpfungsstellen für Integrin als Rezeptor zur Anbindung von Zellen.

Bevorzugt erfolgt die Biofunktionalisierung nach dem Herstellen einer photovernetzten Struktur und der Entfernung des Substrats und cytotoxischer Stoffe wie der Photoinitiator-Komponente und/oder der Stützstruktur durch Nachbehandlung.

In einer bevorzugten Ausführungsform wird das photovernetzbare oder photovernetzte Material bevorzugt über eine Michael-Addition, mit organischen primären Aminen, Gelatine und/oder Thioheparinsulfat funktionalisiert.

In einer bevorzugten Ausführungsform werden mit Thiolgruppen modifizierte Biopolymere, insbesondere Proteine wie Kollagen, Gelatine und Fibronektin oder Polysaccharide wie Cellulose, Stärke, Glycogen, Hyaluronsäure, Chondroitinsulfat, Heparinsulfat und Heparin, insbesondere Heparinsulfat, an die nicht umgesetzten Doppelbindungen der zwei- oder dreidimensionalen Struktur, insbesondere an die Oberfläche dieser Struktur, über eine Thiol-En-Michael-Addition kovalent gebunden. Diese Biofunktionalisierung erfolgt bevorzugt nach jeder Verfahrensfolge der Schritte a) und b).

In einer bevorzugten Ausführungsform werden nicht alle Thiolgruppen des modifizierten Biopolymers über die Thiol-En-Michael-Addition umgesetzt. Bevorzugt werden in einem weiteren Schritt diese nicht umgesetzten Thiolgruppen zumindest teilweise mit bevorzugt Acrylat-modifizierten Biopolymeren, insbesondere Proteine wie Kollagen, Gelatine und Fibronektin oder Polysaccharide wie Cellulose, Stärke, Glycogen, Hyaluronsäure, Chondroitinsulfat, Heparinsulfat und Heparin, insbesondere Heparinsulfat, über eine Thiol-En-Michael-Addition umgesetzt. Die bevorzugt alternierende Umsetzung der hergestellten photovernetzten Struktur mit Thiol-modifizierten Biopolymeren und Acrylat-modifizierten Biopolymeren wird bevorzugt so oft wiederholt, bis die Oberfläche der zwei- oder dreidimensionalen Struktur das modifizierte Biopolymer im gewünschten Anteil beziehungsweise Bedeckungsgrad enthält.

In einer bevorzugten Ausführungsform ist das für die biofunktionelle Komponente verwendete Protein ein Strukturprotein wie Kollagen und/oder ein denaturiertes Protein wie Gelatine.

In einer bevorzugten Ausführungsform ist das für die biofunktionelle Komponente verwendete Glykosamingylkan Heparin, Heparinsulfat, Chondroitinsulfat und/oder Keratansulfat.

In einer bevorzugten Ausführungsform werden, insbesondere in der äußersten Schicht der zwei- oder dreidimensionalen Struktur, biofunktionelle Komponenten, zum Beispiel Adhäsionsanker, insbesondere Cys-RGD (Cystein-Arginin-Glycin-Aspartat), kovalent, insbesondere über eine Thiol-En-Michael-Addition oder durch oxidative Bildung von Disulfidbrücken, gebunden, bevorzugt zur stabilen Adhäsion von Zellen an die Oberfläche der zwei- oder dreidimensionalen Struktur, bevorzugt zur vollständigen Endotheliarisierung.

In einer bevorzugten Ausführungsform ist die biofunktionelle Komponente indirekt über Nanopartikel mit dem photovernetzbaren oder photovernetzten Material verbunden.

In einer bevorzugten Ausführungsform weisen die Nanopartikel molekülspezifische Erkennungsstellen auf. In einer bevorzugten Ausführungsform der vorliegenden Erfindung ist die biofunktionelle Komponente kovalent oder nicht-kovalent an die Nanopartikel gebunden.

In einer bevorzugten Ausführungsform weisen die Nanopartikel in ihrem Inneren die biofunktionelle Komponente auf. In einer bevorzugten Ausführungsform weisen die Nanopartikel in ihrem Inneren Hohlräume auf, wobei in den Hohlräumen die biofunktionelle Komponente vorliegt.

In einer bevorzugten Ausführungsform weisen die Nanopartikel ein polymeres Matrixmaterial auf, wobei die biofunktionelle Komponente mit dem polymeren Matrixmaterial vermischt und gegebenenfalls ionisch gebunden ist.

In einer bevorzugten Ausführungsform wird die verkapselte, biofunktionelle Komponente durch Auflösen des Nanopartikels in einem Lösungsmittel, bevorzugt Wasser, freigesetzt.

In einer bevorzugten Ausführungsform besteht der Nanopartikel aus miteinander kovalent oder nicht-kovalent vernetzten biofunktionellen Komponenten.

In einer bevorzugten Ausführungsform weist die mindestens eine biofunktionelle Komponente mindestens eine funktionelle Gruppe auf, mit der die biofunktionelle Komponente mit den Nanopartikeln verbunden ist, insbesondere mit den molekülspezifischen Erkennungsstellen der Nanopartikel.

In einer bevorzugten Ausführungsform erfolgt die Bindung der biofunktionelle Komponente mit der mindestens einer funktionellen Gruppe an die molekülspezifischen Erkennungsstellen der Nanopartikel, an dem erste funktionelle Gruppen aufweisende molekülspezifische Erkennungsstellen der Nanopartikel mit die ersten funktionellen Gruppen bindenden, komplementären zweiten funktionellen Gruppen aufweisenden biofunktionellen Komponenten derart in Kontakt gebracht werden, dass kovalente und/oder nicht-kovalente Bindungen zwischen den funktionellen Gruppen der molekülspezifischen Erkennungsstellen und der biofunktionellen Komponenten erfolgen.

In einer bevorzugten Ausführungsform sind die ersten funktionellen Gruppen und die die ersten funktionellen Gruppen bindenden komplementären zweiten funktionellen Gruppen ausgewählt aus der Gruppe bestehend aus Aktivester, Alkylketongruppe, Aldehydgruppe, Aminogruppe, Carboxygruppe, Epoxygruppe, Maleinimidogruppe, Hydazingruppe, Hydrazidgruppe, Thiolgruppe, Thioestergruppe, Oligohistidingruppe, Strep-Tag I, Strep-Tag II, Desthiobiotin, Biotin, Chitin, Chitinderivate, Chitinbindedomäne, Metallchelatkomplex, Streptavidin, Streptactin, Avidin und Neutravidin.

In einer bevorzugten Ausführungsform wird eine biokompatible Struktur hergestellt, wobei die polymere Vernetzer-Komponente in einer Menge von 5 bis 80 Masse-%, insbesondere 5 bis 30 Masse-%, und die mindestens eine Photoinitiator-Komponente in einer Menge von 0,2 bis 4 Masse-%, vorzugsweise 0,5 bis 1 Masse-%, bevorzugt weniger als 0,5 Masse-% vorliegt.

In einer bevorzugten Ausführungsform weist das photovernetzbare Material zusätzlich mindestens eine kurzkettige Vernetzer-Komponente mit mindestens drei photovernetzbaren Gruppen auf, die aus der Gruppe ausgewählt sind bestehend aus Acrylat, Methacrylat, Acrylamid, Methacrylamid, Urethanacrylat, Urethanmethacrylat, Ureaacrylat und Ureamethacrylat.

In einer bevorzugten Ausführungsform ist die kurzkettige Vernetzer-Komponente aus der Gruppe ausgewählt bestehend aus kurzkettigen polyfunktionalen Alkoholen und kurzkettigen polyfunktionalen Aminen.

In einer bevorzugten Ausführungsform ist die kurzkettige Vernetzer-Komponente ausgewählt aus der Gruppe bestehend aus Trimethyl-olpropan, Pentaerythrit, Trimethylolpropanpropoxylat, Glycerolpropoxylat, Trimethylolpropan und Di(trimethylolpropan).

In einer bevorzugten Ausführungsform ist die kurzkettige Vernetzer-Komponente mit mindestens drei photovernetzbaren Gruppen ausgewählt aus der Gruppe bestehend aus Trimethylolpropan-triacrylat, Pentaerythrit-triacrylat, Trimethylolpropanpropoxylat-triacrylat, Glycerolpropoxylat-triacrylat, Trimethylolpropan-trimethacrylat, Di(trimethylolpropan)-tetraacrylat und Pentaerythrittetraacrylat.

In einer bevorzugten Ausführungsform weist das photovernetzbare Material zusätzlich mindestens eine niedrig-viskose Modifikatorkomponente mit einer photovernetzbaren Gruppe auf, die aus der Gruppe ausgewählt ist bestehend aus Acrylat, Methacrylat, Acrylamid, Methacrylamid, Urethanacrylat, Urethanmethacrylat, Ureaacrylat und Ureamethacrylat.

In einer bevorzugten Ausführungsform ist die niedrig-viskose Modifikatorkomponente Laurylacrylat und/oder Isobornylacrylat.

In einer bevorzugten Ausführungsform weist das photovernetzbare Material zusätzlich mindestens eine Verdünner-Komponente auf. Die Verdünner-Komponente ist ein wässriges oder organisches Lösungsmittel, das bevorzugt einen hohen Dampfdruck besitzt. Der hohe Dampfdruck des wässrigen oder organischen Lösungsmittels dient zur teilweise oder kompletten, schnellen Verflüchtigung vor der Aushärtung des in Schritt b) photovernetzten Materials.

In einer bevorzugten Ausführungsform ist die Photoinitiator-Komponente aus der Gruppe ausgewählt bestehend aus alpha-Hydroxyketone, alpha-Morpholino-ketone, Phosphinoxide, Campherchinone, N,N,N',N'-substituierte Benzidine, dreifach arylsubstituierte Amine und Diynone.

In einer bevorzugten Ausführungsform ist die Photoinitiator-Komponente aus der Gruppe ausgewählt bestehend aus 1-Hydroxycyclohexylphenylketon, 4-(2-Hydroxyethoxy)phenyl-(2-hydroxy-2-propyl)keton, Phenyl-bis(2,4,6-trimethylbenzoyl)-phosphin-oxid, 2-Hydroxy-2-methyl-1-phenylpropan-1-on, 2-Methyl-1-[4-(methylthio)phenyl]-2-morpholinpropan-1-on, N⁴,N^{4'}-bis(3-methoxy-phenyl)-N⁴,N^{4'}-diphenyl-4,4'-diaminobiphenyl und 1,5-Diphenyl-1,4-diyn-3-on.

In einer bevorzugten Ausführungsform weisen die zur ortsselektiven Fixierung verwendeten photovernetzbaren Materialien mindestens eine Photoinitiator-Komponente auf, die aus der Gruppe ausgewählt sind bestehend aus 1-Hydroxycyclohexylphenylketon, 4-(2-Hydroxyethoxy)phenyl(2-hydroxy-2-propyl)keton und N⁴,N^{4'}-bis(3-methoxyphenyl)-N⁴,N^{4'}-diphenyl-4,4'-diaminobiphenyl.

In einer bevorzugten Ausführungsform weist das photovernetzbare Material eine Photoinitiator-Komponente auf, die ausgewählt ist aus der Gruppe bestehend aus 1-Hydroxy-Cyclohexylphenylketon, 4-(2-Hydroxyethoxy)phenyl-(2-hydroxy-2-propyl)keton, Phenylbis(2,4,6-trimethylbenzoyl)-phoshinoxid, 2-Methyl-1-[4-(methylthio)phenyl]-2-morpholinpropan-1-on, N⁴,N^{4'}-bis(3-Methoxyphenyl)-N⁴,N^{4'}-diphenyl-4,4'-diaminobiphenyl und 1,5-Diphenyl-1,4-diyn-3-on. Diese vorgenannten Photoinitiator-Komponenten sind besonders bevorzugt zur Herstellung einer biokompartiblen zwei- oder dreidimensionalen Struktur verwendbar.

In einer bevorzugten Ausführungsform weist das photovernetzbare Material zusätzlich mindestens eine Stabilisator-Komponente auf, der aus der Gruppe ausgewählt ist bestehend aus Hydrochinone und Monomethyletherhydrochinone, vorzugsweise in einer Menge von weniger als 500 ppm, bevorzugt weniger als 200 ppm, bevorzugt weniger als 100 ppm (bezogen auf die Stoffmenge der in dem photovernetzbaren Material vorhandenen Komponenten). Die Beimengung einer Stabilisator-Komponente verhindert bevorzugt spontane oder thermisch unkontrollierte Polymerisationen des photovernetzbaren Materials.

In einer besonders bevorzugten Ausführungsform weist das photovernetzbare Material mindestens eine polymere Vernetzer-Komponente mit mindestens zwei photovernetzbaren Gruppen, eine kurzkettige Vernetzer-Komponente mit mindestens drei photovernetzbaren Gruppen, eine niedrig-viskose Modifikatorkomponente mit einer photovernetzbaren Gruppe, mindestens eine Photoinitiatorkomponente, eine Komponente zur Biofunktionalisierung und ein wässriges oder organisches Lösungsmittel auf.

In einer bevorzugten Ausführungsform enthält das photovernetzbare Material zusätzlicheine Absorberkomponente, welche, insbesondere für SL-Verfahren, die Strukturauflösung in Strahlrichtung der elektromagnetischen Strahlung erhöht.

In einer bevorzugten Ausführungsform, hier als erste Verfahrensfolge bezeichnet, erfolgt in Schritt a) das Aufbringen des mindestens einen photovernetzbaren Materials auf der Arbeitsebene ortsselektiv und das in Schritt b) durchgeführte Fixieren des mindestens einen in Schritt a) photovernetzbaren Materials durch elektromagnetische Strahlung flächig.

In einer bevorzugten Ausführungsform, hier als zweite Verfahrensfolge bezeichnet, erfolgt in Schritt a) das Aufbringen des mindestens einen photovernetzbaren Materials auf der Arbeitsebene flächig und das in Schritt b) durchgeführte Fixieren des mindestens einen in Schritt a) aufgebrachten photovernetzbaren Materials durch elektromagnetische Strahlung ortsselektiv.

In einer weiteren bevorzugten Ausführungsform, hier als dritte Verfahrensfolge bezeichnet, erfolgt in Schritt a) das Aufbringen des mindestens einen photovernetzbaren Materials auf der Arbeitsebene flächig und das in Schritt b) durchgeführte Fixieren des mindestens einen in Schritt a) aufgebrachten photovernetzbaren Materials durch elektromagnetische Strahlung flächig.

In einer weiteren bevorzugten Ausführungsform, hier als vierte Verfahrensfolge bezeichnet, erfolgt in Schritt a) das Aufbringen des mindestens einen photovernetzbaren Materials auf der Arbeitsebene ortsselektiv und das in Schritt b) durchgeführte Fixieren des mindestens einen in Schritt a) aufgebrachten photovernetzbaren Materials durch elektromagnetische Strahlung ortsselektiv.

In einer bevorzugten Ausführungsform wird die Verfahrensfolge der Schritte a) und b) mindestens zweimal, vorzugsweise mindestens 500 mal, vorzugsweise mindestens 1000 mal, vorzugsweise 2- bis 600 mal, insbesondere 400- bis 600 mal, bevorzugt 500 mal durchgeführt.

In einer bevorzugten Ausführungsform wird das Verfahren so durchgeführt, dass eine, zwei, drei oder alle vier der vorgenannten Verfahrensfolgen allein oder in Kombination miteinander durchgeführt werden, wobei jede einzelne der unterschiedlichen Verfahrensfolgen ein-, mehrmals oder vielmals durchgeführt werden. In einer weiteren bevorzugten Ausführungsform kann auch vorgesehen sein, dass zumindest zwei der vorgenannten Verfahrensfolgen ausgewählt aus der Gruppe der ersten, zweiten, dritten und vierten Verfahrensfolge miteinander kombiniert durchgeführt werden, wobei jede einzelne der unterschiedlichen Verfahrensfolgen ein-, mehrmals oder vielmals durchgeführt werden kann.

In einer bevorzugten Ausführungsform wird in einer ersten Verfahrensfolge in Schritt a) ortsselektiv wenigstens ein erstes photovernetzbares Material auf der Arbeitsebene aufgebracht und in einem Schritt b) dieses flächig durch elektromagnetische Strahlung fixiert, insbesondere zur Herstellung einer Zentimeter oder Millimeter großen, also makroskopischen Struktur, und anschließend in einer zweiten Verfahrensfolge in einem Schritt a) flächig wenigstens ein zweites photovernetzbares Material auf der Arbeitsebene aufgebracht und in einem Schritt b) dieses ortsselektiv durch elektromagnetischen Strahlung fixiert, insbesondere zur Herstellung einer mikro- oder submikrometergroßen Unterstruktur.

In einer bevorzugten Ausführungsform wird die erste Verfahrensfolge der Schritte a) und b) mindestens zweimal, vorzugsweise mindestens 500 mal, vorzugsweise mindestens 1000 mal, vorzugsweise 2 bis 600 mal, insbesondere 400 bis 600 mal, insbesondere 500 mal und die zweite Verfahrensfolge der Schritte a) und b) zusätzlich mindestens 2 mal, vorzugsweise mindestens 500 mal, vorzugsweise mindestens 1000 mal, vorzugsweise 2 bis 600 mal, insbesondere 400 bis 600 mal, insbesondere 500 mal durchgeführt.

In einer bevorzugten Ausführungsform wird die erste Verfahrensfolge der Schritte a) und b) jeweils abwechselnd mit der zweiten Verfahrensfolge der Schritte a) und b) durchgeführt.

In einer weiteren bevorzugten Ausführungsform kann vorgesehen sein, dass in den vorstehend beschriebenen bevorzugten Ausführungsformen, im Rahmen derer eine erste und eine zweite Verfahrensfolge miteinander kombiniert durchgeführt werden, zusätzlich zumindest eine dritte und/oder zumindest eine vierte Verfahrensfolge durchgeführt wird.

In einer weiteren bevorzugten Ausführungsform ist vorgesehen, dass innerhalb einer Verfahrensfolge, insbesondere innerhalb des Verfahrensschritts a) zwei oder mehr unterschiedliche photovernetzbare Materialien, insbesondere ortsselektiv, aufgebracht und anschließend in Schritt b) fixiert werden. In einer weiteren bevorzugten Ausführungsform der vorliegenden Erfindung wird in einer ersten Verfahrensfolge der Schritte a) und b) ein photovernetzbares Material eingesetzt, das sich von einem in einer zweiten Verfahrensfolge der Schritte a) und b) eingesetzten photovernetzbaren Material unterscheidet.

In einer bevorzugten Ausführungsform werden in der Verfahrensfolge der Schritte a) und b) mindestens zwei verschiedene photovernetzbare Materialien mit unterschiedlichen Photoempfindlichkeiten eingesetzt.

In einer bevorzugten Ausführungsform werden in der Verfahrensfolge der Schritte a) und b) mindestens ein photovernetzbares Material und mindestens ein nichtphotovernetzbares Stützmaterial eingesetzt. Das nicht-photovernetzbares Stützmaterial bildet eine Stützstruktur aus.

In einer bevorzugten Ausführungsform werden in der ersten Verfahrensfolge der Schritte a) und b), insbesondere in Kombination mit der zweiten Verfahrensfolge der Schritte a) und b), mindestens zwei verschiedene photovernetzbare Materialien mit unterschiedlichen Photoempfindlichkeiten eingesetzt.

In einer bevorzugten Ausführungsform wird ein Substrat verwendet, auf dem die wenigstens eine Schicht, vorzugsweise eine Vielzahl von Schichten abgeschieden werden. Als Substrat wird ein steifes oder flexibles Substrat verwendet, insbesondere kann das Substrat aus einem Kunststoffmaterial hergestellt sein. In einer besonders bevorzugten Ausführungsform kann das Substrat eine Plastikfolie, Plastikfilm, Membran, Glas, Metall, Halbmetall, Vlies oder Papier sein, vorzugsweise aus biokompatiblem, insbesondere bioabbaubarem Material.

In einer bevorzugten Ausführungsform wird das Substrat im Anschluss an Schritt b), vorzugsweise nach Abschluss einer wiederholten Durchführung der Verfahrensfolgen a) und b), von der erhaltenen zwei- oder dreidimensionalen Struktur abgetrennt, insbesondere durch chemischen, physikalischen oder biologischen Abbau.

In einer bevorzugten Ausführungsform bleibt das Substrat im Anschluss an Schritt b), vorzugsweise nach Abschluss einer wiederholten Durchführung der Verfahrensfolgen a) und b), Teil der hergestellten Struktur und wird so zum integralen Bestandteil der zwei- oder dreidimensionalen Struktur.

In einer bevorzugten Ausführungsform wird die Biokompatibilität durch spezielle Waschprotokolle bereitgestellt.

Insbesondere wird mit polaren und/oder unpolaren organischen Lösungsmitteln und wässrigen Pufferlösungen gewaschen. Insbesondere wird fünf Tage lang täglich mit je 3 mL 70%igem Ethanol (bezogen auf eine Materialoberfläche von 7 cm²) gewaschen. Insbesondere wird das photovernetzte Material während dieses Waschvorgangs bei Raumtemperatur auf einem Schüttler inkubiert. Nach dem Waschvorgang mit Ethanol wird insbesondere zwei Tage lang im Vakuum getrocknet. Nach dem Trocknen wird insbesondere dreimal mit einem PBS-Puffer (PBS = Phosphat buffered saline) gewaschen.

In einer bevorzugten Ausführungsform weist die mit dem erfindungsgemäßen Verfahren herstellbare zwei- oder dreidimensionale Struktur ein E-Modul (ElastizitätsModul) von 0,1 MPa bis 100 MPa, bevorzugt 1 MPa bis 40 MPa, bevorzugt 1 MPa bis 20 MPa, bevorzugt 0,5 MPa bis 10 MPaauf.

In einer bevorzugten Ausführungsform weist die zwei- oder dreidimensionale Struktur eine Quellbarkeit in Wasser von 1% bis 700%, bevorzugt 300% bis 700%, bevorzugt 1% bis 500%, bevorzugt 1% bis 100 %, bevorzugt 1% bis 10 %, insbesondere 0,5% bis 5% auf.

In einer bevorzugten Ausführungsform weist die zwei- oder dreidimensionale Struktur eine Zugfestigkeit (Sigma) von 0,01 MPa bis 10 MPa, bevorzugt 0,1 MPa bis 1 MPa auf.

In einer bevorzugten Ausführungsform weisen die auf der nicht biofunktionalisierten zwei- oder dreidimensionalen Struktur kultivierte Zellen (je nach Anwendung verschiedener Zelltypen) nach 48 Stunden eine Konfluenz von mindestens 10%, bevorzugt mindestens 50%, bevorzugt mindestens 80% auf.

In einer bevorzugten Ausführungsform weisen die auf der biofunktionalisierten zwei- oder dreidimensionalen Struktur kultivierten Zellen (je nach Anwendung verschiedener Zelltypen) nach 48 Stunden eine Konfluenz von mindestens 50 %, bevorzugt mindestens 60 %, bevorzugt mindestens 80 %, bevorzugt mindestens 90 % auf.

In einer bevorzugten Ausführungsform ist die zwei- oder dreidimensionale Struktur eine Matrix für die Besiedelung mit Zellen zur Herstellung eines in vitro oder in vivo Gewebes, eines Organteils oder Organteil-Äquivalents, eines Organs oder Organ-Äquivalents, eines Transplantats, eines Implantats, eines Gefäßes, eines Gefäßsystems, eines Hohlorgans oder eines Teils eines Hohlorgans, eines Zellkultursubstrats, porösen oder nicht-porösen Transportsystemen, porösen oder nicht-porösen Röhrensystemen, porösen oder nicht-porösen Schläuchen, einer Membran, eines diagnostisches Systems oder eines chirurgischen Gerätes, insbesondere eines Endoskops, oder Teiles davon.

In einer bevorzugten Ausführungsform ist die zwei- oder dreidimensionale Struktur ein in vitro oder in vivo Gewebe, ein Organteil oder Organteil-Äquivalent, ein Organ oder Organ-Äquivalent, ein Transplantat, ein Implantat, ein Gefäß, ein Gefäßsystem, ein Hohlorgan oder ein Teil eines Hohlorgans, ein Zellkultursubstrat, poröse oder nicht-poröse Transportsysteme, poröse oder nicht-poröse Röhrensysteme, poröse oder nicht-poröse Schläuche, eine Membran, ein diagnostisches System oder ein chirurgisches Gerät, insbesondere ein Endoskop, oder Teil davon.

In einer bevorzugten Ausführungsform ist das Gefäß oder das Gefäßsystem ein Blutgefäß wie eine Arterie, Vene oder Kapillare, ein Lymphgefäß wie Lymphkapillaren, Kollektoren, Lymphstämme, ein Speichel- oder Tränengang oder ein weiterer Gang für ein Drüsensekret wie Galle, Milch oder Sperma.

In einer bevorzugten Ausführungsform ist das Hohlorgan eine Speiseröhre, ein Magen-Darm-Trakt, eine Gallenblase, eine Luftröhre, ein Herz, ein Eileiter, ein Samenleiter, ein Harnleiter, eine Harnblase oder eine Harnröhre.

Die dreidimensionale Struktur eignet sich bevorzugt für die Verwendung als vaskuläres System. Die dreidimensionale Struktur zeichnet sich daher bevorzugt durch ihre Biokompatibilität, ihre möglichst vollständige Aushärtung zur Vermeidung toxischer monomerer Bestandteile, die Anwesenheit möglichst geringer Photoinitiatormengen mit geringer oder völlig fehlender Toxizität, die Realisierung ausreichender elastischer Eigenschaften im ausgehärteten Material, eine ausreichende mechanische und biologische Langzeitstabilität und eine biofunktionale oder biofunktionalisierbare Oberfläche aus.

Weitere vorteilhafte Ausgestaltungen ergeben sich aus den Unteransprüchen.

### Kurze Beschreibung der Erfindung

Die Erfindung wird nachstehend anhand von Ausführungsbeispielen unter Bezugnahme auf die Zeichnungen exemplarisch beschrieben. Es zeigen:

| | |
|---|---|
| Figur 1 | schematisierte Darstellung der lösungsgemäßen Vorrichtung, |
| Figur 2 | schematisierte Illustration eines Querschnittes durch eine schichtweise aufgebaute Struktur mit Makro- und Mikrostrukturbereichen, |
| Figur 3 | eine schematisierte Darstellung einer Biofunktionalisierung mittels der Thiol-En-Michael-Addition und anschließender ionischen Anbindung von Wachstumsfaktoren und kovalenter Anbindung von Adhäsionsanker, |
| Figur 4 | eine Rasterelektronenmikroskop-Aufnahme eines mittels MPP und des erfindungsgemäß eingesetzten photovernetzbaren Materials hergestellten Kapillargefäß gemäß Beispiel 3, |
| Figur 5 | Mikrostrukturen innerhalb einer Inkjet gedruckten Materialschicht, schematische und rasterelektronenmikroskopische Aufnahme, |
| Figur 6 | Ergebnisse einer Vitalfärbung und |
| Figur 7 | Röhrchen aus erfindungsgemäßem Material. |

### Wege zur Ausführung der Erfindung, gewerbliche Verwendbarkeit

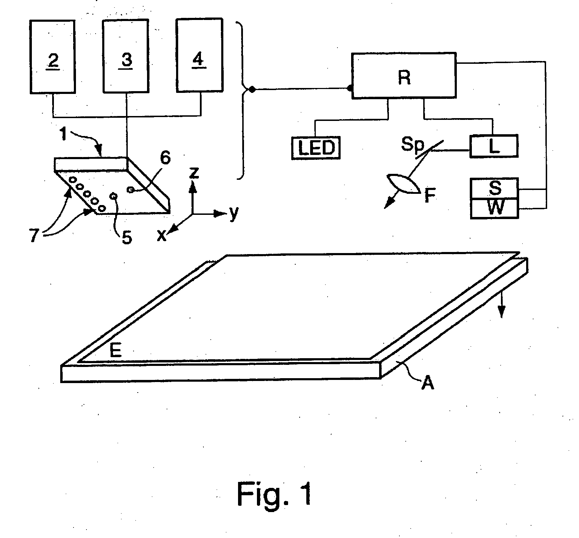

Figur 1 zeigt schematisiert eine Vorrichtung zur schichtweisen Herstellung einer 3D-Struktur mit einer Druckkopfanordnung 1, die mit drei Reservoirbehältern 2, 3, 4 verbunden ist. Im Reservoirbehälter 2 ist ein Stützmaterial, im Reservoirbehälter 3 ein photovernetzbares Material, das gemeinsam mit dem Stützmaterial ortsselektiv mittels der Druckkopfanordnung 1 auf die Arbeitsebene E ausbringbar ist. Hierzu sieht die Druckkopfanordnung 1 wenigstens zwei Druckdüsen 5, 6 vor, durch die das Stützmaterial sowie das photovernetzbare Material ortsselektiv auf der Arbeitsebene E ausbringbar sind. Ferner ist die Druckkopfanordnung 1 mit einem weiteren Reservoirbehälter 4 verbunden, in dem weiteres photovernetzbares Material bevorratet ist, dessen optisches Absorptionsvermögen sich von dem optischen Absorptionsvermögen des photovernetzbaren Materials innerhalb des Reservoirbehälters 3 unterscheidet. Das aus dem Reservoirbehälter 4 stammende photovernetzbare Material dient zum Austrag aus einer längs einer Linearachse angeordneten Vielzahl von Druckdüsen 7, die im gezeigten Ausführungsbeispiel in y-Richtung über die Arbeitsebene E geführt werden. Das durch die Druckdüsen 7 ausgetragene photovernetzbare Material wird als homogene Materialschicht auf der Arbeitsebene E aufgetragen.

Ferner sieht die Vorrichtung in dem gezeigten Ausführungsbeispiel zwei Lichtquellen, nämlich eine Leuchtdiodenanordnung LED sowie eine Laserlichtquelle L vor.. Beide Lichtquellen sind mit einer Regeleinheit R verbunden, die eine entsprechende Aktivierung der Lichtquellen LED, L vornimmt. Der Laserstrahl des Lasers L wird über Ablenkspiegel SP sowie einer optischen Fokussiereinheit F ortsselektiv in eine auf der Arbeitsebene E aufgebrachte Materialschicht fokussiert.

Desweiteren ist eine Messeinrichtung S vorgesehen, das mittels optischer Sensoren die Oberflächenbeschaffenheit die auf die Arbeitsebene A aufgetragene Materialschicht sowie deren Schichtdicke zu erfassen vermag. Desweiteren ist eine Wärmeeinheit W vorgesehen, die zielgerichtet einen Wärmeeintrag auf die Arbeitsebene E und die darauf aufgebrachten Materialdepositionen vornehmen kann. Sowohl die Wärmequelle W als auch die Messeinrichtung S sind mit der Regeleinheit R verbunden. Die Regeleinheit R, die zudem auch eine Steuerfunktion besitzt, steuert bzw. koordiniert sämtliche Komponenten der Vorrichtung, d.h. die Druckkopfanordnung 1 mit den damit verbundenen Reservoirbehältern 2, 3, 4 sowie auch die Strahlenquellenanordnung LED, L mit den damit verbundenen Funktionseinheiten Sp, F.

Zu iterativ schichtweisen Abscheidung entsprechend photovernetzbarer Materialien innerhalb der Arbeitsebene E gilt es einen entsprechenden Arbeitstisch A vorzusehen, der relativ zur Arbeitsebene E vertikal längs zur z-Richtung absenkbar ist.

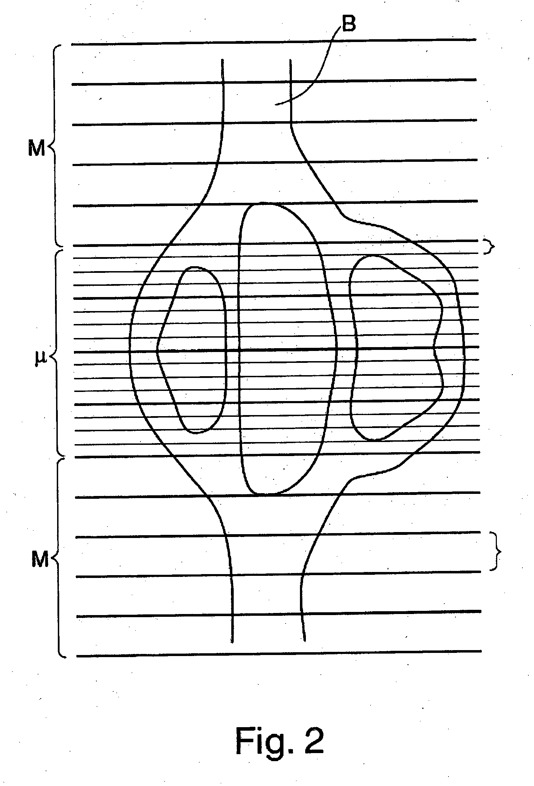

In Figur 2 ist stark schematisiert eine schichtweise aufgebaute Struktur B dargestellt, die sowohl über Makrostrukturbereiche M sowie auch Mikrostrukturbereiche µ aufweist. Die makroskopischen Strukturbereiche M werden mit der 3D Drucktechnik realisiert, bei der ein ortsselektiver Materialauftrag auf der Arbeitsebene mit nachfolgender vollflächiger Ausleuchtung und damit verbundener vollständiger Verfestigung des ortsselektiv ausgebrachten photovernetzbaren Materials erfolgt. Es sei vorausgesetzt, dass das ortsselektiv ausgebrachte photovernetzbare Material einen Photoinitiator einer ersten Art vorsieht. Demgegenüber wird zur Herstellung der Mikro- bzw. Submikrometerstrukturen im Bereich µ das photovernetzbare Material mit einem Photoinitiator einer zweiten Art vollflächig auf der Arbeitsebene ausgebracht und nachfolgend ortsselektiv mit Hilfe eines fokussierten Laserstrahls belichtet.

Die Abfolge bzw. der Übergang von Makrostrukturen M zu Mikrostrukturen µ erfolgt nahtlos und somit einstückig, zumal die Vorrichtung eine sofortige Umschaltung zwischen den zwei beschriebenen Verfahrensvarianten von einer Prozessschicht zur nächsten ermöglicht.

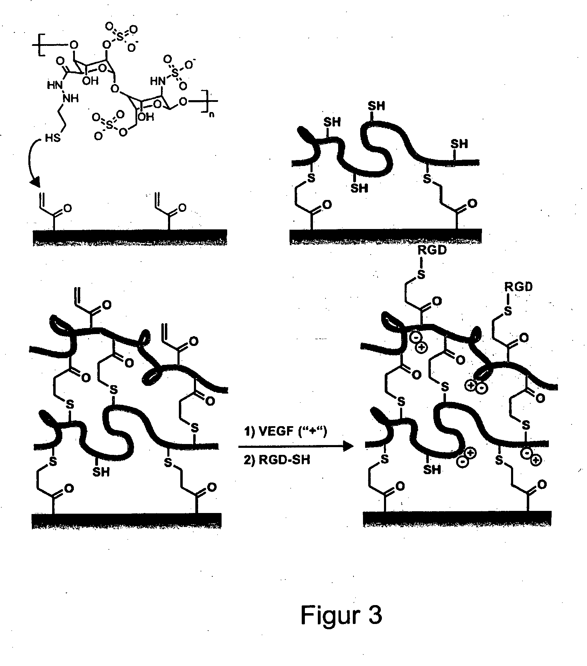

Figur 3 zeigt beispielhaft eine nachträgliche Biofunktionalisierung des fotovernetzten Materials indem in einem ersten Schritt eine Umsetzung der in dem Schritt b), d.h. Bestrahlungsschritt zur Fixierung, nicht umgesetzten Acrylatgruppen eines photovernetzten Materials mit Thiol modifiziertem Heparinsulfat über die Thiol-En-Michael-Addition, wobei ein Teil der Thiolgruppen des modifizierten Heparinsulfats nicht umgesetzt wird. In einem weiteren Schritt werden diese nicht umgesetzten Thiolgruppen teilweise mit einem Acrylat-modifizierten Biopolymer wie Heparin über die Thiol-En-Michael-Addition kovalent gebunden. Die Schritte 1 und 2 werden so oft wiederholt (ist nicht in dieser Figur gezeigt), bis die Oberfläche der zwei- oder dreidimensionalen photovernetzten Struktur das modifizierte Biopolymer im gewünschten Anteil beziehungsweise Bedeckungsgrad enthält. Anschließend wird in einem Schritt 3 an die durch die Sulfat-Gruppen eingeführten negativen Ladungen ionisch VEGF, ein Wachstumsfaktor, und an die freien Acrylat-Gruppen über eine Thiol-En-Michael-Addition RGD-SH, ein Adhäsionsanker, gebunden.

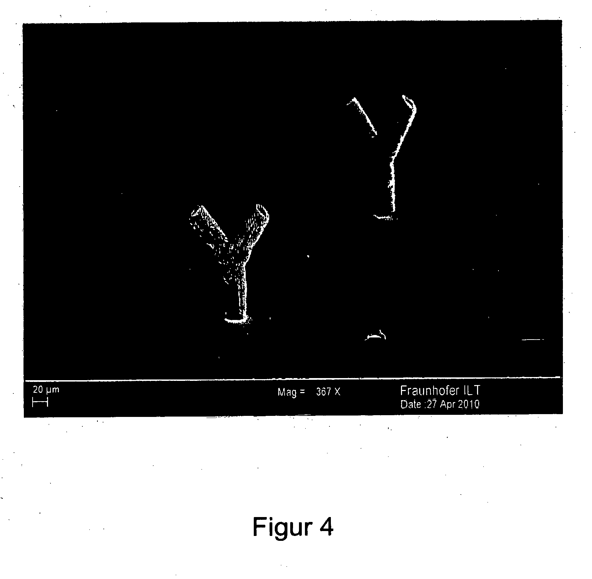

Figur 4 zeigt eine Rasterelektronenmikroskop-Aufnahme eines mittels MPP erzeugten Kapillargefäßes gemäß dem nachstehend erläuterten Beispiel 3.

### Beispiel 1: Synthese von polymeren Vernetzer-Komponenten mit mindestens zwei photovernetzbaren Gruppen

Die Veresterungen von alpha-omega-Dihydroxypolyethern wurden nach den Vorschriften der Druckschrift Toy PH and Janda KD, New supports for solid-phase organic synthesis: development of polystyrene resins containing tetrahydrofuran derived cross-linkers. Tetrahedron Letters. 1999, 40(35), 6329-6332, zu alpha,omega-Dihydroxy-(polyether)-diacrylaten durchgeführt. Die Vorschriften wurden auf die jeweilig eingesetzten Polyether angepasst. Zudem wurden die Vorschriften um einen zusätzlichen Aufreinigungsschritt erweitert, um toxische Katalysatoren und Stabilisatoren aus den Materialien vollständig zu entfernen. Die geklammerte Zahl in den Produktbezeichnungen wie bei "PTHF(1400)-Diacrylat" bezieht sich auf das durchschnittliche Molekulargewicht des eingesetzten Edukts. Die folgende Vorschrift ist als Standardmethode anzusehen. Die Strukturbestimmung der Produkte wurde mit ¹H-NMR durchgeführt. Weiterhin wurden die Produkte mittels GPC, FTIR und gegebenenfalls mit Angabe des R_{f} charakterisiert.

### 1.1 PTHF(1400)-Diacrylat:

40g pTHF (Poly(tetrahydrofuran) - durchschnittliche Mₙ ∼ 1.400, Aldrich, 28,57mmol), 5,08g (4,8ml, 69,3mmol) Acrylsäure, 0,49g (2.5mmol) p-Toluolsulfonsäure-monohydrat und 0,098g (0,1 mmol) Hydrochinon wurden in 600 ml Dichlorbenzol gelöst und in einer Dean-Stark Apparatur 48 Stunden unter Rückfluss erhitzt, bis keine weitere Wasserabscheidung zu beobachten war. Das Reaktionsgemisch wurde anschließend mit 30g K₂CO₃ bei 40 °C drei Stunden gerührt und danach filtriert. Das Filtrat wurde solange mit 10 mM wässriger NaOH-Lösung extrahiert, bis die Wasserphase farblos war. Danach wurde bis zur pH-Neutralisierung mit destilliertem Wasser extrahiert. Es wurde ein leicht gelblicher, viskoser Rückstand erhalten, der im Hochvakuum getrocknet wurde.
¹H-NMR(CDCl₃): δ (ppm) = 1,6 (CH₂, 78H, brs); 3,4 (-O-CH₂, 78H, brs); 4,2 (CH₂-Ac, 4H, t); 5,8 (=CH₂, 2H, d); 6,18 (-CH, 2H, dd); 6,4 (=CH₂, 2H, d)
FTIR: 813, 1100, 1195, 1369, 1727, 2855, 2929;
GPC (THF, Polystyrol (PS)-Standard): Mₙ=1867 g/mol; P=2,4;
R_{f} (Ethylacetat)=0,62.

### 1.2 PTHF(2000)-Diacrylat

PTHF(2000)-Diacrylat wurde hergestellt nach Methode gemäß 1.1.
¹H-NMR(CDCl₃): δ (ppm) = 1,6 (CH₂, 138H, brs); 3,4 (-O-CH₂, 138H, brs); 4,2(CH₂-Ac, 4H, t); 5,8 (=CH₂, 2H, d); 6,18 (-CH, 4H, dd); 6,4 (=CH₂, 2H, d)
FTIR: 813, 1100, 1195,1369,1727,2855,2929;
GPC (THF, PS-Standard): Mₙ=2959 g/mol; P=2,48.

### 1.3 PTHF(2960)-Diacrylat

PTHF(2900)-Diacrylat wurde hergestellt nach Methode gemäß 1.1.
¹H-NMR(CDCl₃): δ (ppm) = 1,6 (CH₂, 166H, brs); 3,4 (-O-CH₂, 162H, brs); 4,2(CH₂-Ac ,4H, t); 5,8 (=CH₂, 2H, d); 6,18 (-CH, 4H, dd); 6,4 (=CH₂, 2H, d)
FTIR: 813, 1100, 1195, 1369, 1727, 2855, 2929;
GPC (THF, PS-Standard): Mₙ=5200g/mol; P=2.33.

### 1.4 PPG(2000)-Diacrylat:

PPG(2000)-Diacrylat wurde hergestellt nach Methode gemäß 1.1. Lediglich Benzol wurde anstatt von Dichlorbenzol als Lösungsmittel verwendet.
¹H-NMR(CDCl₃): 1,08 (brs, CH₃, 68.84H); 3,4 (brs, CH, 22.18H); 3,6 (brs, CH₂, 45.48) 5,1 (q, CH, 1H); 5,8 + 6,12 + 6,4(d,dd,d, 3-CH, 3·1H)
GPC (THF, PS-Standard): Mₙ=66345 g/mol; P=1.15.

### 1.5 PPG(2300)-Diurethan(meth)acrylat:

Toluol-2,4-diisocyanat terminierten Poly(propylenglycol) (Mₙ ∼ 2,300 g/mol) wurde in HEMA (10-facher Überschuss bezogen auf die Moläquivalente) bei maximal 40°C gerührt, bis im IR die für Diisocyanat charakteristische Banden bei 2170cm⁻¹ nicht mehr sichtbar waren. Überschüssiges HEMA wurde im Vakuum bei 60°C destillativ entfernt. Das HEMA kann auch nicht entfernt und zusammen mit PPG(2300)-Diurethan(meth)acrylat in einem erfindungsgemäßen Verfahren eingesetzt werden.
¹H-NMR(CDCl₃): 1,19 (s, CH₃, 22.14); 1,98 (s, CH₃; 2.94); 2,2 (s, Ar-CH₃, 1.27); 3,2-3,6 (brs, O-CH₂, 23.36); 4,35 + 4,42 (dd, CH₂, HEMA); 5,05 (brs, NH, 0.46); 5,60 (s, CH, 1,00); 6.18(s, CH, 1,00).

### 1.6 PTHF(1600)-Diurethan(meth)acrylat:

PTHF(1600)-Diurethan(meth)acrylat wurde nach Methode gemäß 1.5 hergestellt. Es wurde abweichend dazu Toluol-2,4-diisocyanat terminierten Poly(1,4-butanediol) (Mₙ ∼ 1.600 g/mol) als Ausgangsmaterial eingesetzt.
¹H-NMR(CDCl₃): 1,6 (s, CH₂, 19.56); 1,98 (s, CH₃, 2.95); 2,2 (s, Ar-CH₃, 1.27); 3,42 (s, O-CH₂, 19.49); 4,35 + 4,42(dd, CH₂, HEMA); 5,60 (s, CH, 1.00); 6,18 (s, CH, 1.00).

### 1.7 Alle weiteren in den Tabellen 1 a bis 1c verwendeten und mit "*" gekennzeichneten Komponenten sind käuflich erworben worden.

### Beispiel 2: Eigenschaften der erfindungsgemäß eingesetzten Materialen

Die polymeren Vernetzer-Komponenten mit mindestens zwei photovernetzbaren Gruppen wurden bei 40°C mit 0,5 % Irgacure 184 als Photoinitiator und gegebenenfalls mit Nebenkomponenten gemäß Tabellen 1a bis 1c gemischt und die Viskosität bestimmt. Diese photovernetzbaren Materialien wurden mit UV-Licht flächig bestrahlt und fixiert. Nach einem Waschprotokoll wurden WST-Tests (Test zur Quantifizierung der Stoffwechselaktivität von Zellen) und Konfluenztests zur Überprüfung der Biokompatibilität der photovernetzten Materialien durchgeführt. In Zusammenhang mit der vorliegenden Erfindung wird unter dem Begriff "flächig", insbesondere einem flächigen Aufbringen eines photovernetzbaren Materials oder einem flächigen Fixieren eines aufgebrachten photovernetzbaren Materials verstanden, dass das Aufbringen des Materials oder die fixierende Strahlung gleichmäßig über die gesamte zu beschichtende oder fixierende Materialschicht erfolgt. Demgemäß kann ein flächiger Auftrag eines Materials oder ein flächiges Einwirken der Strahlung zur Ausbildung von dreidimensional ausgebildeten beziehungsweise fixierten Schichten führen. Insbesondere aufgrund des flächigen Materialauftrags beziehungsweise flächigen Einwirkens der Strahlung wird das photovernetzbare Material gleichmäßig aufgebracht beziehungsweise fixiert.

In Zusammenhang mit der vorliegenden Erfindung wird unter dem Begriff "ortsselektiv", insbesondere einem ortsselektiven Aufbringen eines photovernetzbaren Materials oder einem ortsselektiven Fixieren eines aufgebrachten photovernetzbaren Materials verstanden, dass das Aufbringen des Material oder die fixierende Strahlung nicht gleichmäßig über die gesamte zu beschichtende oder fixierende Materialschicht erfolgt.

Außerdem wurde der jeweilige E-Modul und die Reißfestigkeit bestimmt.

Die Biokompatibilität wird bevorzugt durch geeignete Waschprotokolle der ausgehärteten Polymere erreicht. Es wurden WST-1 Proliferations-Tests durchgeführt, die allen untersuchten Materialien der Anwendungsbeispiele aus den Tabellen 1a bis 1c die Biokompatibilität bescheinigen. Weiterhin wurde die Interaktion primärer, humaner Endothelzellen - der Zelltyp der das Blutgefäßsystem in vivo auskleidet - mit den zwei- oder dreidimensionalen Strukturen, so wie die Zellmorphologie und Zellfunktionalität untersucht. Zellkonfluenzen von mindestens 10 % bescheinigen zelladhäsive Eigenschaften von nicht-biofunktionalisierten Polymeren. Die Zellfunktionalität vitaler Endothelzellen auf den Materialien wurde durch einen acLDL (acetyliertes Low Density Lipoprotein)-Aufnahme Test und über immunhistochemische Färbungen der spezifischen Marker CD31, vWF und VE-Cadherin belegt.

### Beispiel 3 - Herstellen einer dreidimensionalen Struktur mittels MPP

Das photovernetzbare Material gemäß Nummer 1 aus Tabelle 1a wurde bei Raumtemperatur mit 2% N⁴,N4'-Bis(4-methoxyphenyl)-N4,N4'-diphenyl-4,4'-diaminobiphenyl gemischt und eine gesättigte Lösung hergestellt.. 20 µl des photovernetzbaren Materials wurde als zweite Verfahrensfolge bezeichneten bevorzugten Ausführungsform flächig auf einen Glasobjektträger gegeben, sodass eine Schichtdicke von 170 µm entstand. Dieses photovernetzbare Material wurde mittels ortsselektiver Laserstrahlung mit einer Wellenlänge von 532 nm über Zweiphotonenprozesse ausgehärtet. Zugrunde gelegt wurde dem Verfahren ein CAD Model der herzustellenden dreidimensionalen Struktur, das vertikal in 75 Ebenenschnitte unterteilt war. Jeder dieser 75 Schnitte wurde dabei mit Bahnkurven gefüllt. Der Laserstrahl wurde entsprechend auf diesen vorgegebenen Bahnkurven geführt, so dass durch diese Fixierung die dreidimensionale Struktur, nämlich ein verzweigtes Kapillargefäss mit einem inneren Durchmesser von 20 µm und einer Höhe von 150 µm, erhalten wurde.

Nach Beendigung des Aushärtungsprozesses wurde nicht vernetztes Material durch Eintauchen in Ethanol über einen Zeitraum von 5 min entfernt. Die so entstandene dreidimensionale Struktur wurde im Anschluss mit einer dünnen Goldschicht beschichtet, um mittels eines Rasterelektronenmikroskops ihre Beschaffenheit zu überprüfen (siehe Figur 4).

**Tabelle 1a**

| Prepolymere (photovernetzbare Materialien) | | | | Polymere (photovernetzte Materialien) | | | |
|---|---|---|---|---|---|---|---|
| Nummer | Substanz / Mischungen (w/w) | Viskosität [mPa·s] | | E-Modul [MPa] | Sigma [MPa] | WST [%]¹ / WST_{direkt}² [%] | Konfluenz [%]³ |
| 1 | PTHF(775)-Diacrylat (DA)^{*} | 122 | | 27,5 | 3,5 | 70/45 | 80 |
| 2 | PTHF(1400)-DA | nb | | 10,5 | 1,8 | 80/15 | 20 |
| 3 | PTHF(2000)-DA | nb | | 5,7 | 1,3 | 80/55 | 10 |
| 4 | PTHF(2900)-DA | nb | | 8 | 1 | nb | nb |
| 5 | 50% PTHF(775)-DA / Isobornylacrylat | 35,8 | | 17,3 | 2,5 | 83/56 | 60 |
| 6 | 45,65% PTHF(775)^{*}; 4,35% Trimethylolpropanpropoxylat-triacrylat^{*} / Isobornylacrylat^{*} | 34 | | 23,1 | 3,0 | 72/65 | 20 |
| 7 | 50% PTHF(2900)-DA / Isobornylacrylat | 480 | | 5,47 | 1,8 | 78/45 | 80 |

**Tabelle 1b**

| Prepolymere (photovernetzbare Materialien) | | | | Polymere (photovernetzte Materialien) | | | |
|---|---|---|---|---|---|---|---|
| Nummer | Substanz / Mischungen (w/w) | Viskosität [mPa·s] | | E-Modul [MPa] | Sigma [MPa] | WST[%]¹ / WST_{direkt}² [%] | Konfluenz [%]³ |
| 8 | 60% PPG(900)-DA*/Isobornylacrylat^{*} | 8-12 | | 99,9 | 7,1 | nb | nb |
| 9 | 50% PPG(900)-DA^{*}/ Isobornylacrylat^{*} | 8-12 | | 13,4 | 1,8 | 70/65 | 70 |
| 10 | 40% PPG(900)-DA^{*}/ Isobornylacrylat^{*} | 16 | | 12,4 | 2,2 | 82/60 | 70 |
| 11 | 50% Bisphenol-A-ethoxylat-DA (M=688 g/mol) in 37.5% Isobornylacrylat und 12.5% Laurylacrylat^{*}, Irgacure 184 | 33 | | 21,9 | 3,0 | 79/65 | 60 |
| 12 | 91,3%PTHF(775)-DA / 8,7% Trimethylolpropan propoxylat triacrylat | 122 | | 30 | 2,3 | 70/45 | 80 |
| 13 | 50% Isobornylacrylat; 45,65% PTHF(775)-DA; 4,35% Trimethylolpropan propoxylat triacrylat | 34 | | 21 | 3,1 | nb | nb |

**Tabelle 1c**

| Prepolymere (photovernetzbare Materialien) | | | | Polymere (photovernetzte Materialien) | | | |
|---|---|---|---|---|---|---|---|
| Nummer | Substanz / Mischungen (w/w) | Viskosität [mPa·s] | | E-Modul [MPa] | Sigma [MPa] | WST [%]¹ / WST_{direkt}² [%] | Konfluenz [%]³ |
| 14 | 30% Gelatine-Methacrylat Acrylierungsgrad ∼ 100%, 0,05% Irgacure 2959 | 40 (50°C) | | nb | nb | 100/80 | 90 (Chondrocyten, 7 Tage) |

| | | | | | | | |
|---|---|---|---|---|---|---|---|
| ¹ WST [%]: Stoffwechselaktivität von Endothelzellen, die auf Standard-Zellkultursubstraten aber in Extrakten der zu testenden Materialien kultiviert wurden, bezogen auf in Standardmedium kultivierten Zellen (100%) ² WST direkt [%]:Stoffwechselaktivität von Endothelzellen, die direkt im Kontakt mit den zu testenden Materialien kultiviert wurden, bezogen auf Zellen, die auf Standard-Zellkultürsubstraten kultiviert wurden (100%) ³ Sofern nicht anders angegeben: Endothelzellen nach 48-stündigen Kultivierungsdauer verwendet | | | | | | | |

Tabellen 1a bis 1c: Physikalische und biologische Eigenschaften der Mischungen (nb = nicht bestimmt).

Die Molmasse der unterschiedlichen PTHF-Diacrylate(DA) hat Einfluß auf den E-Modul, wodurch weiche bis sehr weiche Polymere entstehen (Beispiele 1 bis 4).

Die Tabelle 1 listet die Eigenschaften der Kompositionen auf. Die ausgewählten Beispiele erfüllen alle die erfindungsgemäßen Eigenschaften.

### Beispiel 4

### Erzeugung von Mikrostrukturen innerhalb einer Inkjet-gedruckten Materialschicht

Mittels 3D Inkjet-Drucktechnik wurden einfache Muster (z.B. Quadrate) gedruckt. Es wurden 1 - 8 Materialschichten aufgetragen und anschließend in diesen die Strukturierung mittels MPP vorgenommen. Alternativ wurden zunächst eine oder mehrere Materialschichten aufgetragen und vollständig flächig durch UV-Bestrahlung ausgehärtet. Anschließend wurden auf diese Schicht wiederum eine oder mehrere Materialschichten aufgetragen und in diesen mittels MPP eine Strukturierung erzeugt.

Es wurde ein Spectra SL-128 Druckkopf verwendet. Dieser Standard-Druckkopf erzeugt Tropfen mit einer durchschnittlichen Tropfengröße von 80 pl, die im gedruckten Zustand etwa einen Durchmesser von 80 µm bis 100 µm aufweisen. Durch die selektive Vernetzung mittels MPP innerhalb des gedruckten Materials wurden in diesem Beispiel in lateraler Ausdehnung exakte Strukturen mit einer Auflösung < 10 µm erzeugt.

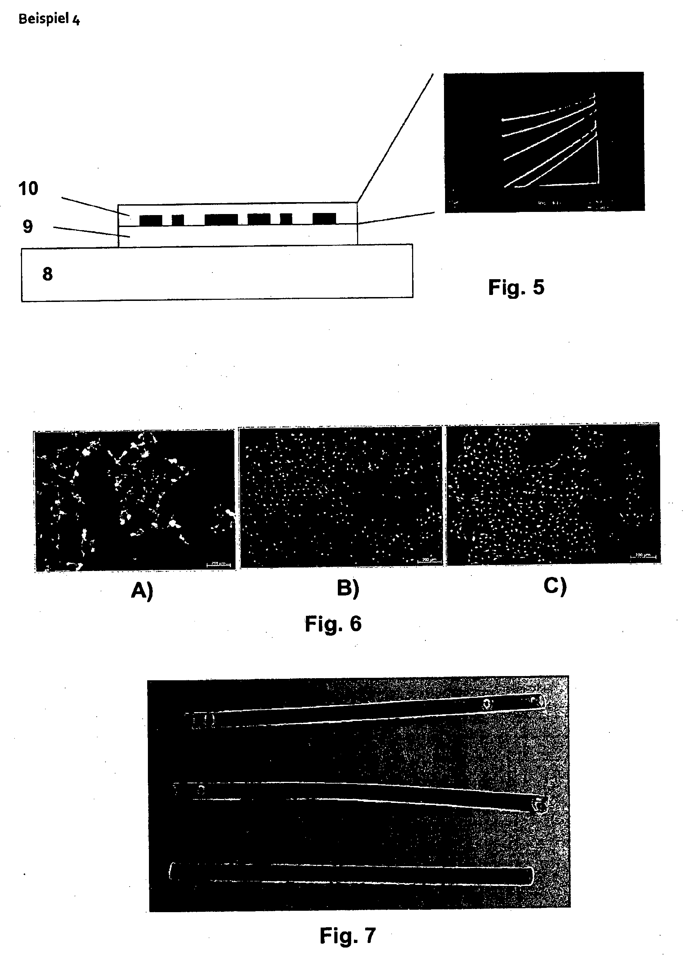

Fig. 5 zeigt die selektive Aushärtung durch MMP innerhalb einer Inkjet-gedruckten Schicht. Links in Figur 5: Schematische Darstellung des zweilagigen Systems. Rechts oben in Figur 5: REM Aufnahme des mikrostrukturierten Materials in einer Druckschicht nach Entfernung des unvernetzten Materials.
Bezugszeichen 8 in Figur 5: Probeträger (Glasslide)
Bezugszeichen 9 in Figur 5: Vollständig flächig ausgehärtete Inkjet-Schicht
Bezugszeichen 10 in Figur 5: Inkjet Schicht mit MPP-Strukturen

### Beispiel 5 - Biofunktionalisierung und Zellbesiedlung der erfindungsgemäßen Materialien

Das als biokompatibel eingestufte Material 11 aus Tabelle 1 wurde mit einer biofunktionalen Schicht aus mit Thiolgruppen funktionalisiertem Heparin (TH) und der Peptid-Sequenz Arginin-Glycin-Asparaginsäure-Cystein (RGDC) beschichtet, um einer thrombogenen Wirkung des Materials entgegenzuwirken und die Adhäsion von Endothezellen zu fördern.

Humane mikrovaskuläre Endothelzellen wurden aus menschlichen Hautbiopsaten isoliert, vorkultiviert und auf die TH-RGDC funktionalisierten Oberflächen, so wie auf Kontrolloberflächen (unfunktionalisiertes Polymer und kommerziell erhältliche Standarzellkulturoberflächen) gegeben. Die Zellen werden für 48h auf den Materialien kultiviert. Danach wird ihre Vitalität, ihre Morphologie, Konfluenz (Zelldichte), metabolische Aktivität und Funktionalität untersucht.

Auf den mit TH und RGDC funktionalisierten Polymeroberflächen sind deutlich höhere Zelldichten und ein wesentlich höherer Anteil an lebendigen Zellen nachweisbar, als auf dem unfunktionalisierten Polymer (Fig. 6). Dies stellt einen eindeutigen Nachweis von Biokompatibilität und Biofunktionalisierbarkeit und Besiedelbarkeit dar.

Fig. 6 zeigt humane mikrovaskulare Endothelzellen (hell und grau) auf A) unfunktionalisiertem erfindungsgemäßem Polymer B) TH-RGDC - funktionalisiertem erfindungsgemäßem Polymer und C) kommerziell erhältlicher Standardzellkultur-Oberfläche. Zellen auf dem funktionalisierten Material (B) zeigen höchste Zelldichte.

Bis auf einzelne wenige Zellen sind alle Zellen lebend und zeigen typische Morphologie. Zellen auf unfunktionalisiertem Material sind lebend, weisen jedoch untypische Morphologie auf und vermehren sich nicht. Zellen auf der Kontroll-Standardzellkulturoberfläche (C) sind bis auf einzelne Zellen lebend und mit typischer Morphologie, Fig. 6A zeigt keine toten Zellen, aber wenig lebende; Fig. 6B und 6C zeigen wenige vereinzelte tote Zellen, viele Zellen sind lebend, in Fig. 6B mehr als in Fig. 6C).

Fig. 7 zeigt Röhrchen aus erfindungsgemäßem Material 11 Tabelle 1. Unten: unfunktionalisiertes Polymer ohne Zellen. Mitte: unfunktionalisiertes Polymer nach Besiedelung mit Endothelzellen und Kultivierung im Bioreaktor. Oben: biofunktionalisiertes Polymer nach Zellbesiedelung und Kultivierung im Bioreaktor. AlamarBlue Nachweis der Zellaktivität: blauer Alamar Blue Farbstoff wird durch vitale Zellen zu einem rot-violetten Farbstoff umgesetzt. Unten: kaum Umsetzung (blau, keine Zellen). Mitte: mittlere Umsetzung (violett, wenige Zellen/geringe Vitalität). Oben: Starke Umsetzung (rot-violett). Zellen auf dem funktionalisierten Material zeigen höchste Vitalität.

### Bezugszeichenliste

- 1: Druckkopfanordnung
- 2, 3, 4: Reservoirbehälter
- 5,6: Druckdüsen
- 7: Linear angeordnete Druckdüsen
- 8: Probeträger (Glasslide)
- 9: Vollständig flächig ausgehärtete Inkjet-Schicht
- 10: Inkjet Schicht mit MPP-Strukturen
- LED: Lichtemittierende Dioden
- L: Laserlichtquelle
- R: Regeleinheit
- W: Wärmeeinheit
- S: Messeinrichtung
- E: Arbeitsebene
- A: Arbeitstisch
- M: Makrostrukturbereich
- µ: Mikrostrukturbereich
- B: Struktur
- SP: Ablenkspiegel
- F: Fokussiereinheit

## Patentansprüche

1. Vorrichtung zur schichtweisen Herstellung von 3D-Strukturen mit einer Druckkopfanordnung (1), die relativ zu einer Arbeitsebene (E) kontrolliert positionierbar ist und mit wenigstens zwei Reservoirbehältern (2, 3, 4) verbunden ist, in denen flüssig bis pastöses photovernetzbares Material mit jeweils unterschiedlichen Photoempfindlichkeiten bevorratet ist, das jeweils über die Druckkopfanordnung (1) in den Bereich der Arbeitsebene (E) ortsselektiv ausbringbar ist, sowie mit einer Strahlungsquellenanordnung, die elektromagnetische Strahlung in Abhängigkeit der Photoempfindlichkeit des ortsselektiv auf die Arbeitsebene (E) ausgebrachten photovernetzbaren Materials flächig emittiert, **dadurch gekennzeichnet, dass** die Strahlungsquellenanordnung wenigstens eine Laserlichtquelle (L) umfasst, deren Laserstrahl mithilfe optischer Strahlablenk- (SP) und Fokussiermittel (F) in einen Bereich einer auf die Arbeitsebene (E) mittels der Druckkopfanordnung (1) flächig ausbringbaren photovernetzbaren Materialschicht fokussierbar ist und im Fokusbereich innerhalb der photovernetzbaren Materialschicht Zweiphotonen- oder Mehrphotonenprozesse, die zur ortsselektiven Verfestigung des photovernetzbaren Materials führen, initiert.

2. Vorrichtung nach Anspruch 1,
**dadurch gekennzeichnet, dass** die Strahlungsquellenanordnung eine die Arbeitsebene (E) flächig bestrahlende erste Strahlungsquelle aufweist, die elektromagnetische Strahlung mit einer ersten Wellenlänge oder einem ersten Wellenlängenspektrum emittiert, und dass
sich die erste Wellenlänge oder das erste Wellenlängenspektrum von der Wellenlänge des Laserstrahls unterscheidet.

3. Vorrichtung nach Anspruch 2,
**dadurch gekennzeichnet, dass** die erste Strahlungsquelle eine Licht emittierende Diode (LED) oder Diodenanordnung oder andere Lichtquelle mit beschränktem Spektralbereich umfasst.

4. Vorrichtung nach Anspruch 1,
**dadurch gekennzeichnet, dass** die Strahlungsquellenanordnung die Laserlichtquelle (L) als einzige Strahlungsquelle umfasst,
dass den Laserstrahl aufweitende optische Mittel vorgesehen sind, durch die der Laserstrahlquerschnitt zur flächigen Beleuchtung des ortsselektiv auf der Arbeitsebene (E) aufgebrachten photovernetzbaren Materials aufweitbar ist, und dass ein optisches Schaltelement vorgesehen ist, das den Laserstrahl entweder den Strahlablenk- und Fokussiermitteln (F) oder dem den Laserstrahl aufweitenden Mittel zuführt.

5. Vorrichtung nach Anspruch 4,
**dadurch gekennzeichnet, dass** ein die Lichtintensität des aufgeweiteten Laserstrahls reduzierendes optisches Mittel im Strahlengang des Laserstrahls eingebracht ist.

6. Vorrichtung nach einem der Ansprüche 1 bis 5,
**dadurch gekennzeichnet, dass** die Druckkopfanordnung (1) zum Ausbringen des photovernetzbaren Materials, das im Wege von Laserstrahl induzierten Zweiphotonen- oder Mehrphotonenprozessen ortsselektiv verfestigbar ist, derart ausgebildet ist, dass das photovernetzbare Material unter Ausbildung einer flächigen Schicht mit einer einheitlichen Schichtdicke und einer planen Schichtoberfläche auf die Arbeitsebene (E) ausbringbar ist.

7. Vorrichtung nach Anspruch 6,
**dadurch gekennzeichnet, dass** die Druckkopfanordnung (1) eine Vielzahl längs einer Linie angeordnete Druckdüsen (7) aufweist, durch die das photovernetzbare Material gleichmäßig ausbringbar ist.

8. Vorrichtung nach einem der Ansprüche 1 bis 7,
**dadurch gekennzeichnet, dass** ein berührungslos arbeitendes Messsystem vorgesehen ist, das die Schichtdicke und/oder die Schichtoberflächenbeschaffenheit der auf der Arbeitsebene (E) abgeschiedenen photovernetzbaren Materialschicht erfasst, und
dass eine Regeleinheit (R) vorgesehen ist, die auf Grundlage eines Soll/Ist-Vergleiches die Druckkopfanordnung (1) im Falle fehlerhafter Schichtdicken und/oder Schichtoberflächenbeschaffenheiten zu Nachkorrekturmaßnahmen ansteuert.

9. Vorrichtung nach Anspruch 8,
**dadurch gekennzeichnet, dass** eine auf die auf der Arbeitsebene (E) abgeschiedene photovernetzbare Materialschicht thermisch einwirkende, von der Regeleinheit (R) ansteuerbare Wärmequelle (W) vorgesehen ist.

10. Vorrichtung nach einem der Ansprüche 1 bis 9,
**dadurch gekennzeichnet, dass** in einer Reservoireinheit (2) Stützmaterial, in wenigstens einer anderen (3) photovernetzbares Strukturmaterial und in wenigstens einer weiteren (4) Reservoireinheit photovernetzbares Material, das im Wege von Laserstrahl induzierten Zweiphotonen- oder Mehrphotonenprozessen ortsselektiv verfestigbar ist, bevorratet sind, und
dass das Stütz- und das photovernetzbare Strukturmaterial über wenigstens zwei unterschiedliche Druckdüsen der Druckkopfanordnung (1) ortsselektiv auf jeweils eine gemeinsame Arbeitsebene (E) ausbringbar sind, und
dass das in der wenigstens einen weiteren Reservoireinheit (4) bevorratete photovernetzbare Material, das im Wege von Laserstrahl induzierten Zweiphotonen- oder Mehrphotonenprozessen ortsselektiv verfestigbar ist, mittels der Druckkopfanordnung (1) in wenigstens einer anderen Arbeitebene (E) flächig ausbringbar ist.

11. Verwendung der Vorrichtung nach Anspruch 10 zur Herstellung makroskopischer Strukturen, die zumindest bereichsweise mikro- oder submikrometer große Unterstrukturen enthalten,
**dadurch gekennzeichnet, dass** die Herstellung der makroskopischen Strukturen (M) durch ortsselektiven Stütz- und Strukturmaterialaustrag auf die Arbeitebene (E) unter Ausbildung lokaler Makrostrukturen (M) erfolgt und die Strahlungsquellenanordnung die Makrostrukturen (M) flächig beleuchtet, und
dass die Herstellung der mikro- oder submikrometer großen Unterstrukturen (µ) durch flächiges Abscheiden des wenigstens einen photovernetzbaren Materials unter Ausbildung einer photovernetzbaren Materialschicht erfolgt, die im Wege von Laserstrahl induzierten Zweiphotonen- oder Mehrphotonenprozessen ortsselektiv verfestigbar ist.

12. Verwendung nach Anspruch 11 zur Herstellung verästelter einstückiger Röhrensysteme mit Röhrendurchmessern und Röhrenlängen, die bereichsweise zwischen 5 mm und 0,1 µm variieren.

13. Verwendung nach Anspruch 11,
**dadurch gekennzeichnet, dass** das Röhrensystem eine poröse Röhrenwand mit Porendurchmesser von 0,1 bis 200 µm, insbesondere 0,5 µm bis 10 µm besitzt.

14. Verwendung nach Anspruch 12 oder 13,
**dadurch gekennzeichnet, dass** die Röhrensysteme aus biologisch kompatiblem Material bestehen oder mit biokompatiblen Material Oberflächig funktionalisiert sind.

15. Verwendung nach einem der Ansprüche 11 bis 14,
**dadurch gekennzeichnet, dass** das Röhrensystem über elastische Eigenschaften verfügt, die den elastischen Eigenschaften von biologischem Gewebe entsprechen.

16. Verfahren zur schichtweisen Herstellung von 3D-Strukturen mit einer Druckkopfanordnung (1), aus der flüssig bis pastöses photovernetzbares Material mit jeweils unterschiedlichen Photoempfindlichkeiten auf eine Arbeitsebene (E) dosiert ausgebracht wird und das jeweils mit einer an die Photoempfindlichkeit des auf die Arbeitsebene ausgebrachten photovernetzbaren Materials abgestimmte elektromagnetische Strahlung flächig beleuchtet wird, wobei sich das ausgebrachte photovernetzbare Material verfestigt,
**dadurch gekennzeichnet, dass** zur Ausbildung einer makroskopischen Struktur (M) aus der Druckkopfanordnung (1) ortsselektiv wenigstens ein erstes photovernetzbares Material als Strukturmaterial auf die Arbeitsebene (E) ausgebracht wird, das zu Zwecken einer Materialverfestigung flächig mit elektromagnetischer Strahlung bestrahlt wird, und
dass zur Ausbildung einer mikro- oder submikrometer großen Unterstruktur (µ) aus der Druckkopfanordnung (1) flächig wenigstens ein zweites photovernetzbares Material unter Ausbildung einer photovernetzbaren Materialschicht auf die Arbeitsebene (E) aufgebracht wird, die ortsselektiv mit einer fokussierten elektromagnetischen Strahlung derart bestrahlt wird, dass im Fokusbereich innerhalb der photovernetzbaren Materialschicht Zweiphotonen- oder Mehrphotonenprozesse, die zur ortsselektiven Verfestigung des zweiten photovernetzbaren Materials führen, initiert werden.

17. Verfahren nach Anspruch 16,
**dadurch gekennzeichnet, dass** gemeinsam mit dem Ausbringen des ersten photovernetzbaren Materials ortsselektiv ein das erste photovernetzbare Material stützendes nicht vernetzendes Stützmaterial mit der Druckkopfanordnung auf die Arbeitsebene (E) ausgebracht wird.

18. Verfahren nach Anspruch 16 oder 17,
**dadurch gekennzeichnet, dass** die elektromagnetische Strahlung zur flächigen Bestrahlung des ersten photovernetzbaren Materials sowie die elektromagnetische Strahlung zur ortsselektiven Bestrahlung des zweiten photovernetzbaren Materials sich bezüglich der Wellenlänge oder des Wellenlängebereiches unterscheiden, oder dass sich die elektromagnetische Strahlung zur flächigen Bestrahlung des ersten photovernetzbaren Materials sowie die elektromagnetische Strahlung zur ortsselektiven Bestrahlung des zweiten photovernetzbaren Materials ausschließlich in der Bestrahlungsintensität unterscheiden.

19. Verfahren nach einem der Ansprüche 16 bis 18,
**dadurch gekennzeichnet, dass** die Verfahrensschritte zum Ausbilden der makroskopischen Struktur (M) sowie zum Ausbilden der mikro- oder submikrometer großen Unterstruktur (µ) jeweils beliebig oft wiederholt und/oder kombiniert werden.

20. Verfahren nach einem der Ansprüche 16 bis 19,
**dadurch gekennzeichnet, dass** die makroskopische Struktur (M) einstückig mit der mikro- oder submikrometer großen Unterstruktur (µ) innerhalb einer Arbeitsebene (E) und/oder schichtweise übereinander verbunden werden.

21. Verfahren nach einem der Ansprüche 16 bis 20,
**dadurch gekennzeichnet, dass** mindestens ein photovernetzbares Material die folgenden Komponenten aufweist:
i) mindestens eine polymere Vernetzer-Komponente mit mindestens zwei photovernetzbaren Gruppen, die aus der Gruppe ausgewählt sind, bestehend aus Acrylat, Methacrylat, Acrylamid, Methacrylamid, Urethanacrylat, Urethanmethäcrylat, Ureaacrylat und Ureamethacrylat und
ii) mindestens eine Photoinitiator-Komponente.

22. Verfahren nach Anspruch 21, wobei die polymere Vernetzer-Komponente aus der Gruppe ausgewählt ist bestehend aus Polyethylenglykol (PEG), Polypropylenglykol (PPG), Siloxane, Polytetrahydrofuran (PTHF), Bis-phenol-A-ethoxylat (BPA-(EO)), Co-Block-Polyether davon, Biopolymere und modifizierte Biopolymere.

23. Verfahren nach Anspruch 21 oder 22, wobei das photovernetzbare Material zusätzlich mindestens eine kurzkettige Vernetzer-Komponente mit mindestens drei photovernetzbaren Gruppen, aufweist, die aus der Gruppe ausgewählt sind, bestehend aus Acrylat, Methacrylat, Acrylamid, Methacrylamid, Urethanacrylat, Urethanmethacrylat, Ureaacrylat und Ureamethacrylat.

24. Verfahren nach Anspruch 23, wobei die kurzkettige Vernetzer-Komponente aus der Gruppe ausgewählt ist bestehend aus kurzkettigen polyfunktionalen Alkoholen und Aminen.

25. Verfahren nach einem der Ansprüche 21 bis 24, wobei das photovernetzbare Material zusätzlich mindestens eine niedrig-viskose Modifikator-Komponente mit einer photovernetzbaren Gruppe aufweist, die aus der Gruppe ausgewählt ist, bestehend aus Acrylat, Methacrylat, Acrylamid, Methacrylamid, Urethanacrylat, Urethanmethacrylat, Ureaacrylat und Ureamethacrylat.

26. Verfahren nach einem der Ansprüche 21 bis 25, wobei die Photoinitiator-Komponente aus der Gruppe ausgewählt ist, bestehend aus alpha-Hydroxyketone, alpha-Morpholinoketone, Phosphinoxide, Campherchinone, N,N,N',N'-substituierte Benzidine, dreifach arylsubstituierte Amine und Diynone.

27. Verfahren nach Anspruch 22, wobei das Biopolymer aus der Gruppe ausgewählt ist, bestehend aus Proteine, Polysaccharide, Glucosaminglykane und Derivate davon.

28. Verfahren nach Anspruch 27, wobei das Protein ausgewählt ist aus der Gruppe bestehend aus Kollagene, Gelatine und Fibronektin.

29. Verfahren nach Anspruch 27, wobei das Polysaccharid ausgewählt ist aus der Gruppe bestehend aus Cellulose, Stärke und Glycogen.

30. Verfahren nach Anspruch 27, wobei das Glukosaminglykan ausgewählt ist aus der Gruppe bestehend aus Hyaluronsäure, Chondroitinsulfat, Heparinsulfat und Heparin.

31. Verfahren nach einem der Ansprüche 21 bis 30, wobei ein Teil der photovernetzbaren Gruppen in dem mindestens einen photovernetzbaren Material im Rahmen der flächigen oder ortsselektiven Bestrahlung nicht umgesetzt wird.

32. Verfahren nach einem der Ansprüche 21 bis 31, wobei das photovernetzbare oder photovernetzte Material mit mindestens einer biofunktionellen Komponente funktionalisiert wird.

33. Verfahren nach Anspruch 32, wobei die mindestens eine biofunktionelle Komponente direkt oder indirekt mit dem photovernetzbaren oder photovernetzten Material verbunden wird.

34. Verfahren nach einem der Ansprüche 31 bis 33, wobei die nicht umgesetzten, photovernetzbaren Gruppen mit mindestens einer biofunktionellen Komponente funktionalisiert werden.

35. Verfahren nach einem der Ansprüche 32 bis 34, wobei die biofunktionelle Komponente ausgewählt ist aus der Gruppe bestehend aus Proteine, Glycoproteine, Wachstumsfaktoren, Antikörper, Peptidsequenzen, Polysaccharide, Glykosaminglykane, Nucleinsäuren, Aptamere und Derivate davon.

36. Verfahren nach einem der Ansprüche 32 bis 35, wobei die biofunktionelle Komponente indirekt über Nanopartikel mit dem photovernetzbaren Material verbunden ist.

37. Verfahren nach Anspruch 36, wobei die biofunktionelle Komponente an die Nanopartikeln kovalent oder nicht-kovalent gebunden ist.

38. Verfahren nach einem der Ansprüche 36 oder 37, wobei die Nanopartikel im Inneren eine biofunktionelle Komponente aufweisen.

39. Verfahren nach einem der vorstehenden Ansprüche 36 bis 38, wobei die Nanopartikel aus miteinander kovalent oder nicht-kovalent vernetzten biofunktionellen Komponenten bestehen.

40. Verfahren nach einem der Ansprüche 21 bis 39, wobei eine biokompatible Struktur hergestellt wird und wobei
- die polymere Vernetzer-Komponente in einer Menge von 5 bis 80 Masse-%, insbesondere 5 bis 30 Masse-% und
- die mindestens eine Photoinitiator-Komponente in einer Menge von 0,2 bis 4 Masse-%, vorzugsweise 0,5 bis 1 % eingesetzt werden.

## Claims

1. A device for the production by layers of 3D structures with a printing head arrangement (1), which can be positioned in a controlled manner relative to a working plane (E) and is connected to at least two reservoir vessels (2, 3, 4), in which liquid to pasty photo-linkable material each with different photo sensitivities is stored, which in each case can be location-selectively output into the region of the working plane (E) via the printing head arrangement (1), and with a radiation source arrangement, which areally emits electromagnetic radiation as a function of the photo sensitivity of the photo-linkable material location-selectively output onto the working plane (E), **characterized in that** the radiation source arrangement comprises at least one laser light source (L), the laser beam of which can be focused with the help of optical radiation deflection (SP) and focussing means (F) in a region of a photo-linkable material layer that can be areally output onto the working plane (E) by means of the printing head arrangement (1) and in the focus region within the photo-linkable material layer, initiates two-photon or multi-photon processes which lead to the location-selective solidification of the photo-linkable material.

2. The device according to claim 1,
**characterized in that** the radiation source arrangement comprises a first radiation source which areally erradiates the working plane (E), which emits electromagnetic radiation with a first wave length or a first wave length spectrum, and **in that** the first wave length or the first wave length spectrum differs from the wave length of the laser beam.

3. The device according to claim 2,
**characterized in that** the first radiation source comprises a light emitting diode (LED) or diode arrangement or other light source with limited spectral range.

4. The device according to claim 1,
**characterized in that** the radiation source arrangement comprises the laser light source (L) as only radiation source,
**in that** optical means expanding the laser beam are provided, through which the laser beam cross section can be expanded for the areal illumination of the photo-linkable material location-selectively applied onto the working plane (E), and
**in that** an optical switching element is provided which feeds the laser beam either to the radiation deflection and focussing means (F) or to the means expanding the laser beam.

5. The device according to claim 4,
**characterized in that** an optical means reducing the light intensity of the expanded laser beam is introduced into the beam path of the laser beam.

6. The device according to any one of the claims 1 to 5, **characterized in that** the printing head arrangement (1) for outputting the photo-linkable material, which by way of laser beam-induced two-photon or multi-photon processes can be location-selectively solidified, is designed in such a manner that the photo-linkable material subject to forming an areal layer with a uniform layer thickness and a planar layer surface can be output onto the working plane (E).

7. The device according to claim 6,
**characterized in that** the printing head arrangement (1) comprises a plurality of printing nozzles (7) arranged alongside a line, through which the photo-linkable material can be uniformly output.

8. The device according to any one of the claims 1 to 7,
**characterized in that** a contactlessly operating measurement system is provided, which detects the layer thickness and/or the layer surface condition of the photo-linkable material layer deposited onto the working plane (E), and
**in that** a regulating unit (R) is provided, which based on a set point/actual comparison actuates the printing head arrangement (1) in the event of defective layer thicknesses and/or layer surface conditions for re-correction measures.

9. The device according to claim 8,
**characterized in that** a heat source (W) that can be activated by the regulating unit (R) and which thermally acts on the photo-linkable material layer deposited onto the working plane (E) is provided.

10. The device according to any one of the claims 1 to 9,
**characterized in that** in a reservoir unit (2) support material, in at least one other one (3) photo-linkable structure material and in at least one further (4) reservoir unit, photo-linkable material, which by way of laser beam-induced two-photon or multi-photon processes can be location-selectively solidified, are stored, and
**in that** the support and the photo-linkable structure material can be location-selectively output via at least two different printing nozzles of the printing head arrangement (1) onto a common working plane (E) each, and
**in that** the photo-linkable material stored in the at least one further reservoir unit (4), which can be location-selectively solidified by way of laser beam-induced two-photon or multi-photon processes, can be areally output in at least one other working plane (E) by means of the printing head arrangement (1).

11. Use of the device according to claim 10 for producing macroscopic structures, which at least in regions contain micro or sub-micrometer sized substructures,
**characterized in that** the production of the macroscopic structure (M) takes place through location-selective support and structure material output onto the working plane (E) subject to forming local macrostructures (M) and the radiation source arrangement areally illuminates the macrostructures (M), and
**in that** the production of the micro or sub-micrometer sized substructures (µ) takes place through areal deposition of the at least one photo-linkable material subject to forming a photo-linkable material layer, which by way of laser beam-induced two-photon or multi-photon processes can be location-selectively solidified.

12. The use according to claim 11 for production of ramified one-piece tube systems with tube diameters and tube lengths which in regions vary between 5 mm and 0.1 µm.

13. The use according to claim 11,
**characterized in that** the tube system has a porous tube wall with pour diameter of 0.1 to 200 µm, in particular 0.5 µm to 10 µm.

14. The use according to claim 12 or 13,
**characterized in that** the tube systems consist of biological compatible material or are functionalised on the surface with bio-compatible material.

15. The use according to any one of the claims 11 to 14,
**characterized in that** the tube system has elastic properties which correspond to the elastic properties of biological tissue.

16. A method for the production by layers of 3D structures with a printing head arrangement (1), from which the liquid to pasty photo-linkable material is output onto a working plane (E) in a metered manner with different photo sensitivities in each case and which in each case is areally illuminated with electromagnetic radiation that is matched to the photo sensitivity of the photo-linkable material output onto the working plane, wherein the output photo-linkable material solidifies,
**characterized in that** for forming a macroscopic structure (M) at least one first photo-linkable material as structure material is location-selectively output from the printing head arrangement (1) onto the working plane (E), which for purposes of material solidification is areally eradiated with electromagnetic radiation, and
**in that** for forming a micro or sub-micrometer sized substructure (µ) at least one second photo-linkable material is areally output from the printing head arrangement (1) onto the working plane (E) subject to forming a photo-linkable material layer, which is location-selectively eradiated with a focussed electromagnetic radiation in such a manner that in the focus region within the photo-linkable material layer two-photon or multi-photon processes, which lead to the location-selective solidification of the second photo-linkable material, are initiated.

17. The method according to claim 16,
**characterized in that** jointly with the outputting of the first photo-linkable material a non-linked support material supporting the first photo-linkable material is location-selectively output with the printing head arrangement onto the working plane (E).

18. The method according to claim 16 or 17,
**characterized in that** the electromagnetic radiation for the areal irradiation of the first photo-linkable material and the electromagnetic radiation for the location-selective irradiation of the second photo-linkable material differ with respect to the wave length or the wave length range, or **in that** the electromagnetic radiation for the areal irradiation of the first photo-linkable material and the electromagnetic radiation for the location-selective irradiation of the second photo-linkable material differ exclusively in the radiation intensity.

19. The method according to any one of the claims 16 to 18,
**characterized in that** the method steps for forming the macroscopic structures (M) and for forming the micro or sub-micrometer sized substructure (µ) in each case can be repeated and/or combined as often as required.

20. The method according to any one of the claims 16 to 19,
**characterized in that** the macroscopic structure (M) are joined in one piece with the micro or sub-micrometer sized substructure (µ) within a working plane (E) and/or by layers on top of one another.

21. The method according to any one of the claims 16 to 20,
**characterized in that** at least one photo-linkable material comprises the following components:
i) at least one polymer cross-linker component with at least two photo-linkable groups which are selected from the group consisting of acrylate, methacrylate, acrylamide, methacrylate, urethane acrylate, urethane methacrylate, urea acrylate and urea methacrylate and
ii) at least one photo-initiator component.

22. The method according to claim 21, wherein the polymer cross-linker component is selected from the group consisting of polyethylene glycol (BEG), polypropylene glycol (PPG), siloxanes, polytetrahydrofuran (PTHF), bisphenol-a-ethoxylate (BBA-(EO)), Co-block polyether thereof, biopolymers and modified biopolymers.

23. The method according to claim 21 or 22, wherein the photo-linkable material additionally comprises at least one short-chain cross-linker component with at least three photo-linkable groups, which are selected from the group consisting of acrylate, methacrylate, acrylamide, methacrylamide, urethane acrylate, urethane methacrylate, urea acrylate and urea methacrylate.

24. The method according to claim 23, wherein the short-chain cross-linker component is selected from the group consisting of short-chain poly-functional alcohols and amines.

25. The method according to any one of the claims 21 to 24, wherein the photo-linkable material additionally comprises at least one low-viscous modifier component with a photo-linkable group that is selected from the group consisting of acrylate, methacrylate, acrylamide, methacrylamide, urethane acrylate, urethane methacrylate, urea acrylate and urea methacrylate.

26. The method according to any one of the claims 21 to 25, wherein the photo initiator component is selected from the group consisting of alpha- hydroxy ketones, alpha-morpholino ketones, phosphine oxides, camphor genomes, N, N, N', N'-substituted benzidines, triple aryl-substituted amines and diynones.

27. The method according to claim 22, wherein the biopolymer is selected from the group consisting of proteins, polysaccharides, glucosaminoglycans and derivatives thereof.

28. The method according to claim 27, wherein the protein is selected from the group consisting of collagens, gelatines and fibronectines.

29. The method according to claim 27, wherein the polysaccharide is selected from the group consisting of cellulose, starch and glycogen.

30. The method according to claim 27, wherein the glucosaminoglycan is selected from the group consisting of hyaluronic acid, chondroitin sulphate, heparin sulphate and heparin.

31. The method according to any one of the claims 21 to 30, wherein a part of the photo-linkable groups in the at least one photo-linkable material is not converted within the scope of the areal or location-selective irradiation.

32. The method according to any one of the claims 21 to 31, wherein the photo-linkable or photo-linked material is functionalised with at least one bio-functional component.

33. The method according to claim 32, wherein the at least one bio-functional component is directly or indirectly connected to the photo-linkable or photo-linked material.

34. The method according to any one of the claims 31 to 33, wherein the unconverted photo-linkable groups are functionalised with at least one bio-functional component.

35. The method according to any one of the claims 32 to 34, wherein the bio-functional component is selected from the group consisting of proteins, glycoproteins, growth factors, antibodies, peptide sequences, polysaccharides, glycosaminoglycans, nucleic acids, aptamers and derivatives thereof.

36. The method according to any one of the claims 32 to 35, wherein the bio-functional component is indirectly connected to the photo-linkable material via nano particles.

37. The method according to claim 36, wherein the bio-functional component is covalently or non-covalently joined to the nano particles.

38. The method according to any one of the claims 36 or 37, wherein the nano particles in the interior comprises a bio-functional component.

39. The method according to any one of the preceding claims 36 to 38, wherein the nano particles consist of bio-functional components which are covalently or non-covalently cross-linked to one another.

40. The method according to any one of the claims 21 to 39, wherein a bio-compatible structure is established and wherein
- the polymer cross-linker component is employed in a quantity of 5 to 80% by mass, in particular 5 to 30% by mass and
- the at least one photo-initiator component is employed in a quantity of 0.2 to 4% by mass, preferentially 0.5 to 1% by mass.

## Revendications

1. Dispositif pour la fabrication par couches de structures 3D, comprenant un agencement de têtes d'impression (1) qui peut être positionné de manière contrôlée par rapport à un plan de travail (E) et est relié à au moins deux réservoirs (2, 3, 4) dans lesquels du matériau photoréticulable liquide à pâteux aux différentes photosensibilités est stocké, qui peut être appliqué par endroits sélectionnés au niveau du plan de travail (E) via l'agencement de têtes d'impression (1), ainsi qu'un agencement de sources de rayonnement qui émet en nappe un rayonnement électromagnétique en fonction de la photosensibilité du matériau photoréticulable appliqué par endroits sélectionnés sur le plan de travail (E), **caractérisé en ce que** l'agencement de sources de rayonnement comprend au moins une source de lumière laser (L) dont le faisceau laser peut être focalisé à l'aide d'un moyen de focalisation (F) et de déviation de faisceau (SP) dans une plage d'une couche de matériau photoréticulable pouvant être appliqué en nappe sur le plan de travail (E) au moyen de l'agencement de têtes d'impression (1) et lance dans la plage de focalisation dans la couche de matériau photoréticulable, des procédés à deux photons ou plus qui ont pour résultat une solidification par endroits sélectionnés du matériau photoréticulable.

2. Dispositif selon la revendication 1,
**caractérisé en ce que** l'agencement de sources de rayonnement présente une première source de rayonnement rayonnant en nappe sur le plan de travail (E), qui émet un rayonnement électromagnétique avec une première longueur d'onde ou un premier spectre de longueur d'onde, et que
la première longueur d'onde ou le premier spectre de longueur d'onde se différencie du faisceau laser.

3. Dispositif selon la revendication 2,
**caractérisé en ce que** la première source de rayonnement comprend une diode émettant de la lumière (LED) ou un agencement de diodes ou une autre source de lumière avec une plage spectrale limitée.

4. Dispositif selon la revendication 1,
**caractérisé en ce que** l'agencement de sources de rayonnement comprend la source de lumière laser (L) en tant qu'unique source de rayonnement,
que des moyens optiques élargissant le faisceau laser sont prévus, à travers lesquels la section transversale du faisceau laser peut être élargie pour l'éclairage en nappe du matériau photoréticulable appliqué par endroits sélectionnés sur le plan de travail (E), et qu'un élément de bascule optique est prévu qui dirige le faisceau laser soit vers les moyens de déviation de faisceau et de focalisation (F) ou le moyen élargissant le faisceau laser.

5. Dispositif selon la revendication 4,
**caractérisé en ce qu'**un moyen optique réduisant l'intensité lumineuse du faisceau laser élargi est inséré dans le trajet du faisceau laser.

6. Dispositif selon l'une des revendications 1 à 5,
**caractérisé en ce que** l'agencement de têtes d'impression (1) pour appliquer le matériau photoréticulable qui peut être solidifié par endroits sélectionnés dans le trajet de procédés à deux photons ou plus induits par faisceau laser, est conçu de telle façon que le matériau photoréticulable peut être appliqué sur le plan de travail (E) par formation d'une couche en nappe avec une épaisseur de couche uniforme et une surface de couche plane.

7. Dispositif selon la revendication 6,
**caractérisé en ce que** l'agencement de têtes d'impression (1) présente une pluralité de buses d'impression (7) disposées le long d'une ligne, à travers lesquelles le matériau photoréticulable peut être appliqué de façon régulière.

8. Dispositif selon l'une des revendications 1 à 7,
**caractérisé en ce qu'**un système de mesure fonctionnant sans contact est prévu, qui détecte l'épaisseur de couche et/ou la nature de surface de la couche de matériau photoréticulable séparée sur le plan de travail (E), et
qu'une unité de réglage (R) est prévue qui commande l'agencement de têtes d'impression (1) pour des mesures de post-correction, en cas d'épaisseurs de et/ou de nature de surface de la couche erronées, en se basant sur une comparaison nominal/réel.

9. Dispositif selon la revendication 8,
**caractérisé en ce qu'**il est prévu une source de chaleur (W) pouvant être commandée par l'unité de réglage (R), agissant de façon thermique sur la couche de matériau photoréticulable séparée sur le plan de travail (E).

10. Dispositif selon l'une des revendications 1 à 9,
**caractérisé en ce que** du matériau de support est stocké dans une unité de réservoir (2), du matériau structurel photoréticulable est stocké dans au moins une autre (3) et dans au moins une unité de réservoir supplémentaire (4) est stocké du matériau photoréticulable qui peut être solidifié par endroits sélectionnés dans le trajet de procédés à deux photons ou plus induits par faisceau laser, et
que les matériaux de support et structurel photoréticulables peuvent être sortis par endroits sélectionnés sur respectivement un plan de travail (E) commun par au moins deux buses d'impression différentes de l'agencement de têtes d'impression (1), et
que le matériau photoréticulable stocké dans l'au moins une autre unité de réservoir (4), qui peut être solidifié par endroits sélectionnés dans le trajet de procédés à deux photons ou plus induits par faisceau laser, peut être sorti en nappe dans l'au moins un autre plan de travail (E) au moyen de l'agencement de têtes d'impression (1).

11. Utilisation du dispositif selon la revendication 10 pour fabriquer des structures macroscopiques qui contiennent au moins par endroits des sous-structures de l'ordre du micromètre ou du sous-micromètre,
**caractérisé en ce que** la fabrication des structures macroscopiques (M) par application par endroits sélectionnés de matériau de support et structurel sur le plan de travail (E) a lieu par formation de macrostructures locales (M) et l'agencement de sources de rayonnement éclaire les macrostructures (M) en nappe, et
que la fabrication des sous-structures (µ) de l'ordre du micromètre ou du sous-micromètre s'effectue par séparation en nappe de l'au moins un matériau photoréticulable par formation d'une couche de matériau photoréticulable, qui peut être solidifiée par endroits sélectionnés dans le trajet de procédés à deux photons ou plus induits par faisceau laser.

12. Emploi selon la revendication 11 pour fabriquer des systèmes de tubes monoblocs ramifiés ayant des diamètres et longueurs de tubes qui varient entre 5 mm et 0,1 µm par endroits.

13. Emploi selon la revendication 11,
**caractérisé en ce que** le système de tubes possède une paroi de tube poreuse ayant un diamètre de pore de 0,1 à 200 µm, en particulier de 0,5 µm à 10 µm.

14. Emploi selon la revendication 12 ou 13,
**caractérisé en ce que** les systèmes de tubes sont en matériau biocompatible ou ont une surface fonctionnalisée avec du matériau biocompatible.

15. Emploi selon l'une des revendications 11 à 14,
**caractérisé en ce que** le système de tubes a des propriétés élastiques qui correspondent aux propriétés élastiques de tissu biologique.

16. Procédé pour la fabrication par couches de structures 3D comprenant un agencement de têtes d'impression (1), duquel du matériau photoréticulable liquide à pâteux aux différentes photosensibilités respectivement, peut être appliqué de façon dosée sur un plan de travail (E) et est éclairé en nappe par un rayonnement électromagnétique déterminé sur la photosensibilité du matériau photoréticulable appliqué sur le plan de travail, sachant que le matériau photoréticulable appliqué se solidifie,
**caractérisé en ce que** pour former une structure macroscopique (M), au moins un premier matériau photoréticulable en tant que matériau structurel est appliqué sur le plan de travail (E) par endroits sélectionnés depuis l'agencement de têtes d'impression (1), lequel est rayonné en nappe avec un rayonnement électromagnétique dans le but de solidifier le matériau, et
que pour former une sous-structure (µ) de l'ordre du micromètre ou du sous-micromètre, au moins un second matériau photoréticulable en tant que matériau structurel est appliqué sur le plan de travail (E) par formation d'une couche de matériau réticulable par endroits sélectionnés depuis l'agencement de têtes d'impression (1), laquelle est rayonnée par endroits sélectionnés avec un rayonnement électromagnétique focalisé de telle façon que dans la plage de focalisation à l'intérieur de la couche de matériau photoréticulable, des procédés à deux photons ou plus qui ont pour résultat une solidification par endroits sélectionnés du second matériau photoréticulable, sont lancés.

17. Procédé selon la revendication 16,
**caractérisé en ce que** conjointement avec l'application du premier matériau photoréticulable par endroits sélectionnés, un matériau support non réticulant supportant le premier matériau photoréticulable est appliqué sur le plan de travail (E) avec l'agencement de têtes d'impression.

18. Procédé selon la revendication 16 ou 17,
**caractérisé en ce que** le rayonnement électromagnétique pour le rayonnement en nappe du premier matériau photoréticulable ainsi que le rayonnement électromagnétique pour le rayonnement par endroits sélectionnés du second matériau photoréticulable se différencient par rapport à la longueur d'onde ou la plage de longueur d'onde, ou que le rayonnement électromagnétique pour le rayonnement en nappe du premier matériau photoréticulable ainsi que le rayonnement électromagnétique pour le rayonnement par endroits sélectionnés du second matériau photoréticulable se différencient exclusivement par l'intensité du rayonnement.

19. Procédé selon l'une des revendications 16 à 18,
**caractérisé en ce que** les étapes de procédé pour former la structure macroscopique (M) ainsi que pour former la sous-structure (µ) de l'ordre du micromètre ou du sous-micromètre sont répétées et/ou combinées aussi souvent que souhaité.

20. Procédé selon l'une des revendications 16 à 19,
**caractérisé en ce que** la structure macroscopique (M) est reliée de façon monobloc à la sous-structure (µ) de l'ordre du micromètre ou du sous-micromètre à l'intérieur d'un plan de travail (E) et/ou par couches l'une sur l'autre.

21. Procédé selon l'une des revendications 16 à 20, **caractérisé en ce qu'**au moins un matériau photoréticulable présente les composantes suivantes :
i) au moins une composante d'agent réticulant polymère comprenant au moins deux groupes photoréticulables sélectionnés parmi le groupe se composant de l'acrylate, du méthacrylate, de l'acrylamide, du méthacrylamide, de l'uréthane-acrylate, de l'uréthane-méthacrylate, de l'urée-acrylate et de l'urée-méthacrylate et
ii) au moins une composante de photoinitiateur.

22. Procédé selon la revendication 21, dans lequel la composante de photoinitiateur est sélectionnée parmi le groupe se composant du polyéthylèneglycol (PEG), du propylèneglycol (PPG), du siloxane, du polytétrahydrofurane (PTHF), du bisphénol-A-éthoxylate (BPA-(EO)), des polyéthers co-blocs de ceux-ci, des biopolymères et des biopolymères modifiés.

23. Procédé selon la revendication 21 ou 22, dans lequel le matériau photoréticulable présente en outre au moins une composante d'agent réticulant à courte chaîne comprenant au moins trois groupes photoréticulables qui sont sélectionnés parmi le groupe se composant de l'acrylate, du méthacrylate, de l'acrylamide, du méthacrylamide, de l'uréthane-acrylate, de l'uréthane-méthacrylate, de l'urée-acrylate et de l'urée-méthacrylate.

24. Procédé selon la revendication 23, dans lequel la composante d'agent réticulant à courte chaîne est sélectionnée parmi le groupe se composant d'alcools et d'amines polyfonctionnels à courte chaîne.

25. Procédé selon l'une des revendications 21 à 24, dans lequel le matériau photoréticulable présente en outre au moins une composante de modificateur à faible viscosité avec un groupe photoréticulable, sélectionné parmi le groupe se composant de l'acrylate, du méthacrylate, de l'acrylamide, du méthacrylamide, de l'uréthanacrylate, de l'uréthane-méthacrylate, de l'urée-acrylate et de l'urée-méthacrylate.

26. Procédé selon l'une des revendications 21 à 25, dans lequel la composante de photoinitiateur est sélectionnée parmi le groupe se composant de l'alpha-hydroxycétone, alpha-morpholinocétone, oxyde de phosphine, campherquinone, benzidine N, N, N', N' substituée, amine ou diynone-aryle substituée trois fois.

27. Procédé selon la revendication 22, dans lequel le biopolymère est sélectionné parmi le groupe se composant de protéine, polysaccharide, glycosaminoglycane et des dérivés de ceux-ci.

28. Procédé selon la revendication 27, dans lequel la protéine est sélectionnée parmi le groupe se composant du collagène, de la gélatine et de la fibronectine.

29. Procédé selon la revendication 27, dans lequel le polysaccharide est sélectionné parmi le groupe se composant de la cellulose, de l'amidon et du glycogène.

30. Procédé selon la revendication 27, dans lequel le glycosaminoglycane est sélectionné parmi le groupe se composant de l'acide hyaluronique, du sulfate de chondroïtine, du sulfate d'héparine et de l'héparine.

31. Procédé selon l'une des revendications 21 à 30, dans lequel une partie des groupes photoréticulables n'est pas convertie en l'au moins un matériau photoréticulable dans le cadre du rayonnement en nappe ou par endroits sélectionnés.

32. Procédé selon l'une des revendications 21 à 31, dans lequel le matériau photoréticulable ou réticulé est fonctionnalisé avec au moins une composante biofonctionelle.

33. Procédé selon la revendication 32, dans lequel l'au moins une composante biofonctionnelle est reliée directement ou indirectement au matériau photoréticulable ou réticulé.

34. Procédé selon l'une des revendications 31 à 33, dans lequel les groupes photoréticulables non convertis sont fonctionnalisés avec au moins une composante biofonctionnelle.

35. Procédé selon l'une des revendications 32 à 34, dans lequel la composante biofonctionnelle est sélectionnée parmi le groupe se composant de protéines, glycoprotéines, facteurs de croissance, anticorps, séquences peptidiques, polysaccharides, glycosaminoglycanes, acides nucléiques, aptamères et des dérivés de ceux-ci.

36. Procédé selon l'une des revendications 32 à 35, dans lequel la composante biofonctionnelle est reliée indirectement au matériau photoréticulable par des nanoparticules.

37. Procédé selon la revendication 36, dans lequel la composante biofonctionnelle est reliée de façon covalente ou non-covalente aux nanoparticules.

38. Procédé selon l'une des revendications 36 ou 37, dans lequel les nanoparticules présentent une composante biofonctionnelle intérieure.

39. Procédé selon l'une des revendications 36 à 38, dans lequel les nanoparticules se composent de composantes biofonctionnelles réticulées de covalente ou non-covalente entre elles.

40. Procédé selon l'une des revendications 21 à 39, dans lequel une structure biocompatible est fabriquée et dans lequel
- la composante d'agent réticulant polymère est employée dans une quantité de 5 à 80 % en masse, en particulier 5 à 30 % en masse et
- l'au moins une composante de photoinitiateur est employée dans une quantité de 0,2 à 4 % en masse, de préférence 0,5 à 1 %.
